# EUROPÄISCHE PATENTSCHRIFT

(11) **EP 0 394 194 B1**
(45) Veröffentlichungstag und Bekanntmachung des Hinweises auf die Patenterteilung: **18.08.1993**
(21) Anmeldenummer: 90810289.0
(22) Anmeldetag: 10.04.1990
(51) Int. Cl.: C07C 237/06, C07C 237/22, C07C 271/22, C07K 1/04, C07K 7/06, C07K 7/08

(54) **Geschützte Aminosäuren und Verfahren zu ihrer Herstellung**
Protected amino-acids and process for their preparation
Aminoacides protégés et procédé pour leur préparation

(30) Priorität: 17.04.1989 CH 1439/89; 20.06.1989 CH 2300/89
(43) Veröffentlichungstag der Anmeldung: 24.10.1990
(73) Patentinhaber: CALBIOCHEM-NOVABIOCHEM AG, CH-4448 Läufelfingen (CH)
(72) Erfinder: Sieber, Peter, CH-4153 Reinach (CH); Riniker, Bernhard, Dr., CH-4402 Frenkendorf (CH)
(74) Vertreter: Monsch, René

(56) Entgegenhaltungen:
- EP-A- 0 292 228
- DE-A- 1 768 047
- DE-B- 1 065 424
- HOUBEN WEYL, "Methoden der organischen Chemie", 4. Auflage, Band XV/1, "Synthese von Peptiden", 1974, Georg Thieme Verlag, Stuttgart, DE, Seiten 711-727

## Beschreibung

Die Erfindung betrifft N-Trityl-Carbamoylgruppen enthaltende Aminosäurederivate, Verfahren zu ihrer Herstellung und ihre Verwendung in der Peptidsynthese.

Die Amidgruppen der Aminosäuren Asparagin und Glutamin führen bei der Peptidsynthese häufig zu unerwünschten Nebenreaktionen, z.B. Dehydratisierung zum Nitril, intramolekulare Cyclisierung zum Imid und, im Falle von Glutamin, zur Pyrrolidonbildung. Ausserdem beobachtet man bei der Festphasensynthese mit Glutamin oft, dass die Peptidharze wegen intermolekularer Ausbildung von Wasserstoffbrücken nicht mehr quellen, was schlechte Kupplungsausbeuten zur Folge hat.

Diese Nachteile können durch Schutz der Carbamoylgruppen von Asparagin und Glutamin vermieden werden. Gebräuchlicherweise verwendet man 4,4'-Dimethoxy-diphenyl-methyl (Di-[4-methoxy-phenyl]-methyl, 4,4'-Dimethoxy-benzhydryl, abgekürzt Mbh) oder 2,4,6-Trimethoxy-benzyl (abgekürzt: Tmob) als Schutzgruppe.

Nun wurde jedoch von den Erfindern der vorliegenden Erfindung gefunden, dass die bisher bekannten Carbamoyl-Schutzgruppen bei ihrer Abspaltung in bestimmten Fällen selbst unerwünschte Nebenreaktionen verursachen. Die bei der acidolytischen Abspaltung gebildeten Carbokationen reagieren nämlich sehr leicht und in irreversibler Weise mit Tryptophan.

Ausgehend von diesem Befund, der als ihr Ausgangspunkt gewissermassen Teil der vorliegenden Erfindung ist, bestand die Aufgabe der vorliegenden Erfindung nun darin, eine Carbamoyl-Schutzgruppe zu finden, bei der die genannten Nebenreaktionen nicht auftreten, und entsprechend geschützte Aminosäurederivate herzustellen.

Die Erfindung betrifft Verbindungen der Formel I, 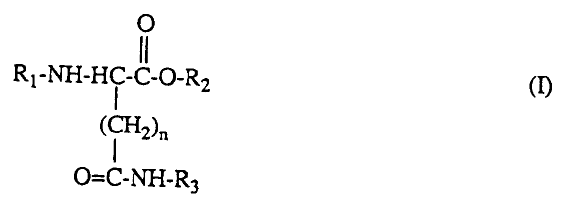
worin n für 1 oder 2 steht, R₁ Wasserstoff oder eine Aminoschutzgruppe, R₂ Wasserstoff oder eine Carboxylschutzgruppe und R₃ Triphenylmethyl, 4-Monomethoxy-trityl oder 4,4'-Dimethoxy-trityl bedeuten, Salze von solchen Verbindungen worin R₂ Wasserstoff ist, mit einer salzbildenden Gruppe und reaktionsfähige Carbonsäurederivate von solchen Verbindungen der Formel I, worin R₂ für Wasserstoff steht und ausserdem R₁ eine Aminoschutzgruppe ist. Die Konfiguration am asymmetrischen HC-Atom ist (D,L), (D) oder vorzugsweise (L).

Die Verbindungen der Formel I sind bei der Peptidsynthese ohne alkylierende Wirkung auf die Indolseitenkette von Tryptophan. Sie sind ausserdem leichter zugänglich, besser löslich in organischen Lösungsmitteln und ihre Amidschutzgruppe kann leichter mit Trifluoressigsäure abgespalten werden als in analogen Verbindungen, worin R₃ für 2,4,6-Trimethoxy -benzyl (Tmob) oder Di-(4-methoxy-phenyl)-methyl (Mbh) steht. Zum Beispiel betragen die Halbwertszeiten von Fmoc-Gln(Trt)-OH, Fmoc-Gln(Tmob)-OH und Fmoc-Gln(Mbh)-OH bei der Spaltung in Trifluoressigsäure-1,2-Dichlorethan (1:1) bei 22°C 2, 9 beziehungsweise 27 Minuten.

Die reaktionsfähigen Carbonsäurederivate der Verbindungen der Formel I sind unter Kupplungsbedingungen, das heisst in Gegenwart von Kupplungskatalysatoren, wie 1-Hydroxy-1H-benztriazol (HOBt), und in Gegenwart von Lösungsmitteln, die üblicherweise für Kupplungsreaktionen bei der Peptidsynthese verwendet werden, z.B. Dimethylacetamid (DMA) und 1,2-Dichlor-ethan (DIEA), deutlich stabiler als analoge Verbindungen, worin R₃ Wasserstoff bedeutet. Zum Beispiel betragen die Zerfall-Halbwertszeiten von Fmoc-Asn-O-Tcp und Fmoc-Asn-O-Pfp (Fmoc = 9-Fluorenyl-methoxycarbonyl, Pfp = Pentafluor-phenyl, Tcp = 2,4,5-Trichlor-phenyl) in DMA + 1 Aequivalent HOBt + 1,7 Aequivalente DIEA nur 2 Minuten beziehungsweise sogar weniger als 1 Minute, während die analogen Verbindungen, worin R₃ einen Tritylrest bedeutet, unter den gleichen Bedingungen vollkommen stabil sind.

Ueberraschenderweise verlangsamen die raumfüllenden Tritylschutzgruppen die Kupplungsreaktionen nicht, sondern haben praktisch keinen Einfluss auf die Kupplungsgeschwindigkeit.

Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis", John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Eine geschützte Aminogruppe R₁-NH kann z.B. in Form einer leicht spaltbaren Acylamino-, Arylmethylamino-, verätherten Mercaptoamino-, 2-Acyl-niederalk-1-enyl-amino- oder Silyl-aminogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Iod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitrobenzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Weitere, als Aminoschutzgruppen R₁ in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-(phenylniederalkyl)-phosphoryl, z.B. Dibenzylphosphoryl oder Di-(4-nitrobenzyl)-phosphoryl, gegebenenfalls substituiertes Phenyloxyphenyl-phosphonyl, z.B. Phenyloxyphenyl-phosphonyl, Diniederalkylphosphinyl, z.B. Diethylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylaminogruppe darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 2-Nitrophenylthio.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäure halbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Ethoxycarbonyl-prop-1-en-2-yl.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, oder Allyloxycarbonyl und diesem verwandte Schutzgruppen, oder vor allem 9-Fluorenyl-methoxycarbonyl.

Carboxylschutzgruppen R₂ sind üblicherweise veresternde Gruppen, in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Methoxybenzyloxycarbonyl oder 4-Nitrobenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, 2-Trimethylsilylethoxycarbonyl oder 2-(Di-n-butyl-methylsilyl)-ethoxycarbonyl, oder 2-Triarylsilylethoxycarbonyl, wie 2-Triphenylsilylethoxycarbonyl.

Bevorzugte geschützte Carboxylgruppen sind tert.-Niederalkoxycarbonyl, wie tert.- Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, und vor allem 2-(Trimethylsilyl)-ethoxycarbonyl.

Eine salzbildende Gruppe in einer Verbindung der Formel I ist z.B. eine freie Aminogruppe (R₁ = H) oder eine freie Carboxylgruppe (R₂ = H). Die Verbindungen der Formel 1, worin R₁ Wasserstoff bedeutet, können Säureadditionssalze bilden, z.B. mit anorganischen Säuren, wie Salzsäure, Schwefelsäure oder Phosphorsäure, oder mit geeigneten organischen Carbon- oder Sulfonsäuren, z.B. Trifluoressigsäure. Die Verbindungen der Formel I, worin R₂ Wasserstoff bedeutet, können Metall- oder Ammoniumsalze bilden, z.B. Akalimetall- oder Erdalkalimetallsalze, z.B. Natrium-, Kalium-, Magnesium- oder Calciumsalze, oder Salze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triethylamin, Hydroxyniederalkylamine, z.B. 2-Hydroxyethylamin, Bis-(2-hydroxyethyl)-amin, 2-Hydroxy-ethyl-diethyl-amin oder Tri-(2-hydroxyethyl)-amin, basische aliphatische Ester von Carbonsäuren, z.B. 4-Amino-benzoesäure-2-diethylaminoethylester, Niederalkylenamine, z.B. 1-Ethylpiperidin, Cycloakylamine, z.B. Dicyclohexylamin, oder Benzylamine, z.B. N,N'-Dibenzylethylendiamin, ferner Basen vom Pyridintyp, z.B. Pyridin, Collidin oder Chinolin.

Verbindungen der Formel I, worin R₁ und R₂ Wasserstoff bedeuten, können innere Salze bilden.

Diese Salze können unter anderem zur Isolierung oder Reinigung der Verbindungen der Formel I verwendet werden, worin R₁ und/oder R₂ Wasserstoff bedeuten.

Reaktionsfähige Carbonsäurederivate einer Verbindung der Formel I, worin R₁ eine Aminoschutzgruppe ist und R₂ für Wasserstoff steht, sind in erster Linie reaktionsfähige aktivierte Ester oder reaktionsfähige Anhydride, ferner reaktionsfähige cyclische Amide.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronenanziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlorphenol, 2,3,4,5,6-Pentafluorphenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. duch Nitro, substituierte Phenyl-thioester (die man z.B. durch Behandeln der entsprechenden Säure mit gegebenenfalls, z. B. durch Nitro, substituierten Thiophenolen, u.a. mit Hilfe der Anhydrid- oder Carbodiimid-Methode, erhalten kann; Methode der aktivierten Thiolester), Amino-oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-5-norbornen-2,3-dicarboximid, N-Hydroxy-piperidin, N-Hydroxy-phthalimid, 1-Hydroxy-benztriazol oder 3,4-Dihydro-3-hydroxy-4-oxo-1,2,3-benzotriazin, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann und die leicht mit Hydroxy-, nicht aber mit Aminogruppen reagieren).

Anhydride von Säuren können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azid methode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkanoder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol erhalten kann; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Die in diesem Text verwendeten Abkürzungen für die Aminosäuren sind die allgemein üblichen. So bedeuten Asp Asparaginsäure, Asn Asparagin, Gln Glutamin, Ser Serin, Leu Leucin, Gly Glycin, Lys Lysin, His Histidin, Pro Prolin, Tyr Tyrosin, Trp Tryptophan, Arg Arginin, Val Valin und Ile Isoleucin.

In Uebereinstimmung mit den international anerkannten Nomenklaturregeln bezeichnen in diesem Text die Abkürzungen für die Aminosäuren, z.B. die obengenannten Abkürzungen, die freie Säure und, wenn nicht anders angegeben, die L-Konfiguration. Die α-Aminogruppe ist an der linken Seite der Abkürzung, die Carboxygruppe an der rechten Seite zu denken. Das Fehlen eines H-Atoms in der α-Amino-Gruppe wird durch einen links an der Abkürzung für die Aminosäure stehenden Bindestrich, das Fehlen von zwei H-Atomen durch zwei links stehende Bindestriche gekennzeichnet. Das Fehlen einer HO-Gruppe in der Carboxygruppe wird durch einen rechts stehenden Bindestrich ausgedrückt. Substituenten in der Seitenkette von Aminosäuren werden unmittelbar hinter das Aminosäuresymbol in Klammern gesetzt. Somit steht z.B. Z-Asn(Trt)-OH für die Verbindung der Formel Ia.

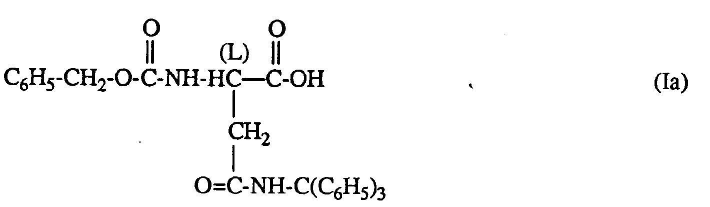

Die durch Verwendung der Verbindungen der Formel I bei der Peptidsynthese erzielten Vorteile sind im Beispielteil illustriert. So wird z.B. H-Gln-Gln-Gln-Gln-Gln-Ser-Leu-Gly-OH als Rohprodukt in einer Reinheit von 92 % erhalten, wenn die Carbamoylgruppe der Gln-Reste bei der Herstellung durch Trityl geschützt wird. Ohne diesen Schutz bei der Merrifield-Synthese enthält das Rohprodukt nicht 92 %, wie oben, sondern nur 30 % des gewünschten Produkts.

Die erhaltenen Rohprodukte aus den Synthesen von Fmoc-Lys-Gln-His-Asn-Pro-Lys-Tyr-Gln-Trp-Asn-OH mit Trityl-Schutz der Carbamoylgruppen von Asn und Gln (Rohprodukt A) einerseits und mit 2,4,6-Trimethoxy-benzyl-Schutz (Rohprodukt B) andererseits weisen etwa 95 % des gewünschten Produkts in Rohprodukt A und nur etwa 8 % des gewünschten Produkts in Rohprodukt B auf.

Die Verbindungen der Formel I können als Bausteine bei der Synthese von beliebigen Peptiden, die Glutamin- und/oder Asparaginreste enthalten, oder als Zwischenprodukte zur Herstellung dieser Bausteine verwendet werden. Zur Peptidsynthese nach Merrifield verwendet man diejenigen Verbindungen der Formel I, worin R₁ eine Aminoschutzgruppe, wie insbesondere 9-Fluorenyl-methoxycarbonyl oder tert.-Butyloxycarbonyl, R₂ Wasserstoff und R₃ Triphenylmethyl, 4-Monomethoxy-trityl oder 4,4'-Dimethoxy-trityl bedeuten, und reaktionsfähige Carbonsäurederivate davon.

Bevorzugt sind Verbindungen der Formel I, worin R₁ Benzyloxycarbonyl, 9-Fluorenylmethoxycarbonyl, Allyloxycarbonyl oder tert.Butyloxycarbonyl, R₂ Wasserstoff und R₃ Triphenylmethyl bedeuten.

Besonders bevorzugt sind die in den Beispielen beschriebenen Verbindungen der Formel I.

Von den oben näher beschriebenen reaktionsfähigen Carbonsäurederivaten sind besonders die 2,4,5-Trichlor-phenylester, die Pentafluor-phenylester, die Ester mit 1-Hydroxybenztriazol, mit 3,4-Dihydro-3-hydroxy-4-oxo-1,2,3-benzotriazin oder mit N-Hydroxy-5-norbornen-2,3-dicarboximid, die Säurechloride und die symmetrischen Anhydride zu erwähnen, insbesondere solche reaktionsfähige Carbonsäurederivate der vorstehend als bevorzugt herausgestellten Verbindungen der Formel I.

Ueberraschenderweise ist die erfindungsgemässe Amidschutzgruppe R₃ stabil bei der katalytischen Hydrierung unter Bedingungen, unter denen Benzyloxycarbonyl oder R₃ als Aminoschutzgruppen oder Benzyl als Carboxylschutzgruppe bereits abgespalten werden. Die Schutzgruppe R₃ kann z.B. mit Trifluoressigsäure-Wasser-1,2-Ethandithiol (90:5:5) im Temperaturbereich zwischen Raumtemperatur und +50°C, z.B. bei +30°C, abgespalten werden.

Die Verbindungen der Formel I werden in an sich bekannter Weise hergestellt. Das erfindungsgemässe Herstellungsverfahren ist dadurch gekennzeichnet, dass man
a) eine Verbindung der Formel II, 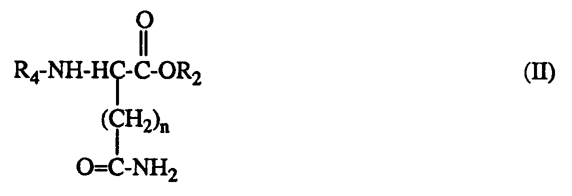 worin R₄ für Wasserstoff oder eine unter den Reaktionsbedingungen stabile Aminoschutzgruppe steht und n und R₂ die obengenannten Bedeutungen haben, mit einer Verbindung der Formel III,

   R₃-OH (III)

   worin R₃ die obengenannte Bedeutung hat, umsetzt und aus dem erhaltenen Produkt diejenigen Schutzgruppen abspaltet, die nicht Bestandteil des gewünschten Endprodukts der Formel I sind, oder
b) zur Herstellung einer Verbindung der Formel I, worin R₁ für eine Aminoschutzgruppe steht, in eine Verbindung der Formel IV, 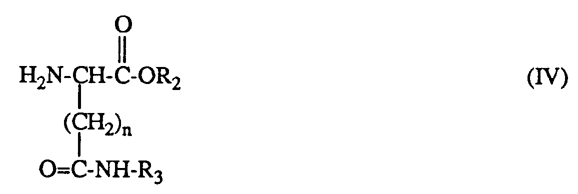 worin n, R₂ und R₃ die obengenannten Bedeutungen haben, eine Aminoschutzgruppe einführt, oder
c) eine Verbindung der Formel V, 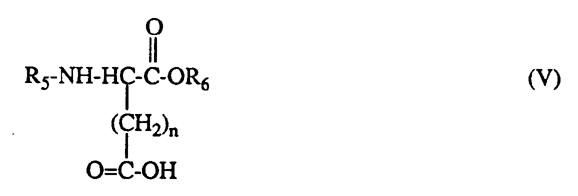 worin n die obengenannte Bedeutung hat, R₅ eine unter den Reaktionsbedingungen stabile Aminoschutzgruppe bedeutet und R₆ für eine unter den Reaktionsbedingungen stabile Carboxylschutzgruppe steht, mit einer Verbindung der Formel VI,

   R₃-NH₂ (VI)

   worin R₃ die obengenannte Bedeutung hat, umsetzt und aus dem erhaltenen Produkt diejenigen Schutzgruppen abspaltet, die nicht Bestandteil des gewünschten Endprodukts der Formel I sind, und, wenn erwünscht, nach Durchführung eines der Verfahren a), b) oder c) eine erhaltene Verbindung der Formel I mit einer salzbildenden Gruppe in ihr Salz überführt oder ein erhaltenes Salz in die freie Verbindung überführt oder eine erhaltene Ver bindung der Formel I, worin R₂ R₁ eine Aminoschutzgruppe und Wasserstoff bedeutet, in ein reaktionsfähiges Carbonsäurederivat überführt.

Die obengenannten Verfahrensvarianten werden im folgenden näher erläutert:

### Verfahren a):

Verfahren a) wird in einem geeigneten organischen Lösungsmittel, wie z.B. Essigsäure, in Gegenwart katalytischer Mengen einer wasserfreien starken Lewis-Säure, wie z.B. Bortrifluorid, Trifluormethansulfonsäure oder vorzugsweise Schwefelsäure, sowie in Gegenwart eines wasserentziehenden Mittels, wie z.B. Acetanhydrid, im Temperaturbereich zwischen etwa 0°C und +100°C, vorzugsweise zwischen Raumtemperatur (etwa 20°C) und +70°C, z.B. bei +50°C durchgeführt. Wenn R₄ Wasserstoff bedeutet, verwendet man zweckmässigerweise mehr wasserfreie Schwefelsäure als im Fall, wo R₄ eine Aminoschutzgruppe bedeutet, z.B. 1,1 Aequivalente, und führt die Reaktion vorzugsweise bei etwa +60°C durch, besonders, wenn man von Verbindungen der Formel II ausgeht, worin R₄ für Wasserstoff und n für 1 stehen.

Eine Aminoschutzgruppe R₄ ist eine unter sauren Bedingungen stabile Aminoschutzgruppe, wie z.B. Allyloxycarbonyl, Trifluoracetyl oder vorzugsweise Benzyloxycarbonyl oder 9-Fluorenyl-methoxycarbonyl.

Wenn im gewünschten Endprodukt der Formel I R₁ und/oder R₂ für Wasserstoff stehen, müssen die Schutzgruppen R₄ und/oder R₂ abgespalten werden.

Die meisten Carboxylschutzgruppen R₂ werden bereits unter den Reaktionsbedingungen abgespalten. Stabil sind lediglich Carboxylschutzgruppen R₂ wie Methyl, Ethyl oder Allyl, die z.B. durch basenkatalysierte Hydrolyse abgespalten werden können.

Trifluoracetyl als Aminoschutzgruppe R₄ wird durch milde alkalische Hydrolyse, z.B. mit wässriger Ammoniaklösung, Natrium- oder Bariumhydroxid oder mit basischen Ionenaustauscherharzen abgespalten.

Benzyloxycarbonyl als Aminoschutzgruppe R₄ wird durch Hydrierung in Gegenwart eines Palladium-auf-Kohle-Katalysators in wasserhaltiger, methanolischer, salzsaurer Lösung bei Raumtemperatur und Normaldruck abgespalten.

9-Fluorenyl-methoxycarbonyl als Aminoschutzgruppe R₄ wird mittels Piperidin-Dimethylacetamid (1:4) bei Raumtemperatur abgespalten.
Die Ausbeuten bei Verfahren a) betragen etwa 60-95 % der Theorie.

### Verfahren b):

Verfahren b) wird in einem geeigneten, gegebenenfalls wasserhaltigen organischen Lösungsmittel oder Lösungsmittelgemisch im Temperaturbereich zwischen etwa 0°C und +80°C, vorzugsweise bei Raumtemperatur bis +50°C, durchgeführt, wobei auch in Suspension gearbeitet werden kann. So kann man z.B. 9-Fluorenyl-methoxycarbonyl als Aminoschutzgruppe R₁ einführen, indem man eine Ausgangsverbindung der Formel IV in einem organischen Lösungsmittel, wie Tetrahydrofuran oder Dioxan, in Gegenwart von Wasser und einer anorganischen Base, wie Natriumhydrogencarbonat oder Natriumhydroxid, sowie erforderlichenfalls von einem tertiären organischen Amin, wie Triethylamin, mit 9-Fluorenylmethyl-N-succinimidyl-carbonat umsetzt. Tert.-Butyloxycarbonyl als Aminoschutzgruppe R₁ kann man z.B. in 90%igem wässrigen Tetrahydrofuran in Gegenwart von Triethylamin bei Raumtemperatur mit äquimolaren Mengen Di-tert.-butyl-dicarbonat einführen. Die Ausbeuten bei Verfahren b) betragen etwa 90-99 % der Theorie.

Im Unterschied zu Verfahren a) gestattet Verfahren b) die Einführung beliebiger Aminoschutzgruppen R₁.

### Verfahren c):

Verfahren c) wird in einem geeigneten organischen Lösungsmittel, wie 1,2-Dichlor-ethan, im Temperaturbereich zwischen -20°C und +70°C, z.B. bei 0°C, und vorzugsweise unter Schutzgas, z.B. unter Argonatmosphäre, durchgeführt. Hierzu ist eine in situ Aktivierung der freien Carboxylgruppe im Ausgangsmaterial der Formel V, z.B. mit Hilfe von 1-Chlor-N,N,2-trimethyl-prop-1-enylamin, erforderlich. Die Ausbeuten bei Verfahren c) betragen etwa 40-70 % der Theorie.

Eine unter den Reaktionsbedingungen stabile Aminoschutzgruppe R₅ ist z.B. 9-Fluorenyl-methoxycarbonyl. Eine unter den Reaktionsbedingungen stabile Carboxylschutzgruppe R₆ ist z.B. Benzyl.

Die Abspaltung der Carboxyl- und/oder Aminoschutzgruppen, die nicht Bestandteil des gewünschten Endprodukts der Formel I sind, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig, wobei auch enzymatische Methoden verwendet werden können.

Insbesondere kann eine Benzylgruppe R₆ durch Hydrierung in Gegenwart eines Palladium-auf-Kohle-Katalysators bei Raumtemperatur abgespalten werden, wobei 9-Fluorenyl-methoxycarbonyl R₅ und die Schutzgruppe R₃ erhalten bleiben. Die Abspaltung von 9-Fluorenyl-methoxycarbonyl R₅ kann wie bei Verfahren a) beschrieben ausgeführt werden. Die Ausbeuten bei der Abspaltung der Schutzgruppen betragen etwa 90-99 % der Theorie.

Vorzugsweise stellt man die Verbindungen der Formel I nach den Verfahren b) und vor allem a) her.

Zusatzoperationen: Salze von Verbindungen der Formel I mit salzbildenden Gruppen können in an sich bekannter Weise hergestellt werden. So kann man Salze von Verbindungen der Formel I mit sauren Gruppen durch Umsetzung mit einer geeigneten Base, z.B. durch Behandeln mit geeigneten Metallverbindungen, wie Alkalimetallsalzen von geeigneten organischen Carbonsäuren, z.B. dem Natriumsalz der α-Ethyl-capronsäure, oder mit geeigneten anorganischen Alkali- oder Erdalkalimetallsalzen, insbesondere solchen, die sich von einer schwachen und vorzugsweise flüchtigen Säure ableiten, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche z.B. eine freie Carboxylgruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden. Die Salzbildung verläuft praktisch quantitativ.

Salze können in üblicher Weise praktisch quantitativ in die freien Verbindungen überführt werden, Metall- und Ammoniumsalze z.B. durch Behandeln mit geeigneten Säuren, und Säureadditionssalze z.B. durch Behandeln mit einem geeigneten basischen Mittel.

Die Herstellung der reaktionsfähigen Carbonsäurederivate einer Verbindung der Formel I, worin R₁ eine Aminoschutzgruppe und R₂ Wasserstoff bedeutet, erfolgt, wie bereits oben, bei der näheren Charakterisierung der reaktionsfähigen Carbonsäurederivate, beschrieben ist. Hierzu kann man z.B. von einer Verbindung der Formel I, worin R₁ eine geeignete Aminoschutzgruppe und R₂ Wasserstoff bedeuten, ausgehen. Die Ausbeuten an reaktionsfähigem Carbonsäurederivat betragen etwa 85-98 % der Theorie.

Die oben beschriebenen Verfahren, inklusive die Verfahren zur Abspaltung von Schutzgruppen und die zusätzlichen Verfahrensmassnahmen, werden, wenn nicht anders angegeben, in an sich bekannter Weise, z.B. in An- oder Abwesenheit von vorzugsweise inerten Lösungs- und Verdünnungsmitteln, wenn notwendig, in Anwesenheit von Kondensationsmitteln oder Katalysatoren, bei erniedrigter oder erhöhter Temperatur, z.B. in einem Temperaturbereich von etwa -20°C bis etwa 150°C, insbesondere von etwa 0°C bis etwa +70°C, vorzugsweise von etwa +10°C bis etwa +50°C, hauptsächlich bei Raumtemperatur, in einem geeigneten Gefäss und erforderlichenfalls in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Die Erfindung betrifft auch die Verwendung der Verbindungen der Formel I, insbesondere die Verwendung derjenigen Verbindungen der Formel I, worin R₁ eine Aminoschutzgruppe, wie Benzyloxycarbonyl, Allyloxycarbonyl, tert.-Butyloxycarbonyl oder insbesondere 9-Fluorenyl-methoxycarbonyl, R₂ Wasserstoff und R₃ 4-Monomethoxytrityl, 4,4'-Dimethoxyaityl oder insbesondere Triphenylmethyl (Trityl) bedeuten, sowie die Verwendung der reaktionsfähigen Carbonsäurederivate dieser Verbindungen zur Synthese von Peptiden, insbesondere an fester Phase, wie z.B. zur Merrifield-Synthese.

Die Erfindung betrifft auch Substanzen, die mindestens einen bivalenten Rest der Formel VII, 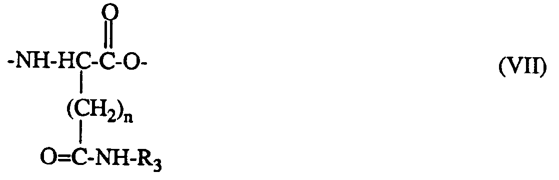
worin n für 1 oder 2 steht und R₃ Triphenylmethyl, 4-Monomethoxy-trityl oder 4,4'-Dimethoxy-trityl bedeutet, enthalten, Verfahren zu ihrer Herstellung und ihre Verwendung zur Synthese von Peptiden. Bei diesen Substanzen handelt es sich in erster Linie um Peptidderivate, wie sie zum Beispiel bei der Peptidsynthese als Zwischenprodukte anfallen. Dementsprechend können diese Peptidderivate auch an ein Syntheseharz gebunden sein.

Die nachfolgenden Beispiele illustrieren die Erfindung, ohne sie einzuschränken. Die Temperaturen sind in Grad Celsius angegeben.

### Abkürzungen

- But: = tert.-Butyl
- Boc: = tert.-Butoxycarbonyl
- Bzl: = Benzyl
- bzw.: = beziehungsweise
- Dhbt: = 3,4-Dihydro-3-hydroxy-4'-oxo- 1,2,3-benzotriazinyl
- FAB-MS: = Fast atom bombardment Massenspektrum
- Fmoc: = 9-Fluorenyl-methoxycarbonyl
- HPLC: = Hochdruckflüssigkeitschromatographie
- konz.: = konzentriert
- Pfp: = Pentafluor-phenyl
- Pmc: = 2,2,5,7,8-Pentamethyl-chroman-6-sulfonyl
- Smp.: = Schmelzpunkt
- Tcp: = 2,4,5-Trichlor-phenyl
- THF: = Tetrahydrofuran
- Tmob: = 2,4,6-Trimethoxy-benzyl
- Trt: = Trityl (Triphenyl-methyl)
- Z: = Benzyloxycarbonyl

### Beispiel 1: Z-Asn(Trt)-OH

Ein Gemisch von 13,3 g Z-Asn-OH (50 mMol), 26 g Triphenylmethanol (100 mMol), 150 ml Essigsäure und 9,5 ml (100 mMol) Acetanhydrid wird bei 50° 15 Minuten gerührt. Nach Zugabe von 0,25 ml konz. Schwefelsäure entsteht nach 5 Minuten eine klare, gelbe Lösung. Nach 1¹/₂ Stunden wird abgekühlt und in 1,5 Liter Eiswasser ausgegossen. Der Niederschlag wird abfiltriert, gut mit Wasser gewaschen und in etwa 500 ml Essigsäureethylester gelöst. Die organische Phase wird mit einer gesättigten wässrigen Natriumchloridlösung gewaschen, über Natriumsulfat getrocknet und unter vermindertem Druck auf 250 g eingeengt. Das Z-Asn(Trt)-OH wird durch Zugabe von 125 ml Hexan zum Kristallisieren gebracht, Smp. 195-196°.

### Beispiel 2: H-Asn(Trt)-OH

Eine Suspension von 10,2 g Z-Asn(Trt)-OH (20 mMol) in 120 ml Methanol und 20 ml einnormaler wässriger Chlorwasserstoffsäure wird in Gegenwart von 0,5 g eines 10%igen Palladium-auf-Kohle-Katalysators bei Raumtemperatur und Normaldruck hydriert. Nach beendeter Wasserstoffaufnahme wird der Katalysator abfiltriert, mit Methanol ausgewaschen und das Filtrat unter vermindertem Druck auf ein Gewicht von 100 g eingeengt. Nach Zusatz von 2,8 ml Triethylamin (20 mMol) wird weiter auf ein Gewicht von 50 g eingeengt, der kristalline Niederschlag wird abfiltriert und mit einem 1:1-Gemisch von Methanol und Wasser, dann mit Wasser chloridfrei gewaschen. Das H-Asn(Trt)-OH enthält 0,5 Mol Wasser und zersetzt sich langsam ab 220°.

### Beispiel 3: Fmoc-Asn(Trt)-OH

Eine Lösung von 3,8 g H-Asn(Trt)-OH · 0,5 H₂O (10 mMol) in 20 ml Tetrahydrofuran, 10 ml Wasser und 1,4 ml Triethylamin (10 mMol) wird unter kräftigem Rühren mit 3,4 g 9-Fluorenylmethyl-N-succinimidyl-carbonat (10 mMol) versetzt und gleichzeitig 1,4 ml Triethylamin so zugetropft, dass der pH-Wert 8,3-8,7 beträgt. Nach 20 Minuten wird im Eisbad gekühlt, mit Essigsäureethylester und 25 ml einer einmolaren wässrigen Kaliumhydrogensulfatlösung versetzt. Die organische Phase wird mit Wasser sulfatfrei gewaschen, über Natriumsulfat getrocknet und unter vermindertem Druck auf etwa 50 g eingeengt. Durch langsames Zugeben von Diisopropylether kann das Fmoc-Asn(Trt)-OH kristallisiert werden, Smp. 210-211° (unter Zersetzung).

### Beispiel 4: Fmoc-Asn(Trt)-OH

Eine Suspension von 3,54 g Fmoc-Asn-OH (10 mMol) und 5,2 g Triphenylmethanol (20 mMol) in 30 ml Essigsäure und 1,9 ml Acetanhydrid (20 mMol) wird 15 Minuten bei 50° gerührt, dann mit einer Lösung von 0,05 ml konz. Schwefelsäure in 5 ml Essigsäure versetzt. Nach etwa einer Stunde bei 50°C bildet sich eine Lösung, und nach 1½ Stunden beginnt sich ein Niederschlag zu bilden. Nach 3 Stunden wird die Suspension unter Eiskühlung langsam mit 200 ml eiskaltem Wasser versetzt, kurz gerührt, der Niederschlag abfiltriert und gut mit Wasser gewaschen. Das feuchte Material wird in Essigsäureethylester gelöst, die organische Phase mit einer gesättigten wässrigen Natriumchloridlösung gewaschen, über Natriumsulfat getrocknet und unter vermindertem Druck eingeengt. Bevor das Produkt auszukristallisieren beginnt wird mit 1,2-Dichlorethan verdünnt, wieder eingeengt und durch 50 g Kieselgel filtriert. Das überschüssige Triphenylmethanol wird mit 200 ml 1,2-Dichlorethan eluiert, dann wird mit je 500 ml eines 98:2- und eines 96:4-Gemisches von 1,2-Dichlorethan und Methanol Fmoc-Asn(Trt)-OH eluiert. Nach Eindampfen zur Trockne wird der Rückstand in 10 ml Tetrahydrofuran gelöst, mit 30 ml Essigsäureethylester versetzt, auf 20 g eingeengt und durch Zusatz von Diisopropylether kristallisiert; das so erhältliche Fmoc-Asn(Trt)-OH schmilzt bei 210-211° (unter Zersetzung).

### Beispiel 5: Fmoc-Asn(Trt)-OH

Eine Lösung von 0,69 g Fmoc-Asn(Trt)-OBzl in 10 ml eines 9:1-Gemisches von Methanol und Wasser wird in Gegenwart von 0,07 g eines 10%igen Palladium-auf-Kohle-Katalysators bei Raumtemperatur und Normaldruck hydriert, bis im Dünnschichtchromatogramm (Lösungsmittel: 1:1 Gemisch von Toluol und Aceton) kein Ausgangsmaterial mehr sichtbar ist. Nach Abfiltrieren des Katalysators wird das Filtrat eingedampft, der Rückstand in einem 96:4-Gemisch von 1,2-Dichlorethan und Methanol gelöst und durch wenig Silikagel filtriert. Nach Eindampfen des Filtrats erhält man das Fmoc-Asn(Trt)-OH, Smp. 210-211° (unter Zersetzung).

Das Ausgangsmaterial kann wie folgt erhalten werden: Stufe 5.1:1,12 g H-Asp-OBzl (5 mMol) und 1,05 g Natriumhydrogencarbonat werden in 25 ml eines 9:1-Gemisches aus Dioxan und Wasser suspendiert, 1,85 g 9-Fluorenylmethyl-N-succinimidyl-carbonat fest zugegeben und das Gemisch 15 Stunden bei Raumtemperatur gerührt. Nach Einengen der Lösung wird mit Essigsäureethylester verdünnt, die organische Phase mit 0,5-molarer wässriger Kaliumhydrogensulfatlösung und Wasser gewaschen, dann über Natriumsulfat getrocknet und eingeengt. Nach Zusatz von Petrolether kristallisiert Fmoc-Asp-OBzl aus.

Stufe 5.2: Eine Lösung von 2,09 g Fmoc-Asp-OBzl in 20 ml 1,2-Dichlorethan wird unter Rühren bei 0° und unter einer Argonatmosphäre mit 0,8 ml 1-Chlor-N,N,2-trimethylprop-1-enylamin (A. Devos et al., J. Chem. Soc., Chem. Commun., Bd. 1979, S. 1180) versetzt. Nach 10 Minuten wird eine Lösung von 1,2 g Tritylamin in 4 ml 1,2-Dichlorethan zugegeben, dann 2 Stunden gerührt, mit Essigsäureethylester verdünnt und mit Wasser gewaschen. Die organische Phase wird getrocknet und eingedampft. Der Rückstand wird in Chloroform aufgenommen und an 250 g Silikagel chromatographiert. Die den Fmoc-Asn(Trt)-OBzl enthaltenden Fraktionen werden vereinigt und eingedampft; das Produkt wird als amorpher Schaum erhalten.

### Beispiel 6: Z-Gln(Trt)-OH

Ein Gemisch von 2,8 g Z-Gln-OH (10 mMol), 5,2 g Triphenylmethanol (20 mMol) in 30 ml Essigsäure wird mit 1,1 ml Essigsäureanhydrid (12 mMol) versetzt und 15 Minuten bei 50° gerührt. Dann werden 0,05 ml konz. Schwefelsäure (etwa 1 mMol) zugegeben, wobei in wenigen Minuten eine klare, gelbe Lösung entsteht. Nach 2 Stunden wird die Lösung unter Rühren und Eiskühlung in 500 ml Wasser eingetropft, der Niederschlag abfiltriert und gut mit Wasser gewaschen. Das feuchte Material wird in Essigsäureethylester gelöst, die wässrige Phase abgetrennt, die organische Phase einmal mit einer gesättigten wässrigen Natriumchloridlösung gewaschen, über Natriumsulfat getrocknet und unter vermindertem Druck auf 20 g eingeengt. Durch portionenweise Zugabe von Hexan wird das Z-Gln(Trt)-OH kristallisiert, Smp. 161-162°.

### Beispiel 7: H-Gln(Trt)-OH

Eine Suspension von 10,4 g Z-Gln(Trt)-OH (20 mMol) in 180 ml eines 9:1-Gemisches von Methanol und Wasser und 20 ml einnormaler wässriger Chlorwasserstoffsäure wird bei Raumtemperatur und Normaldruck in Gegenwart von 0,5 g eines 10%igen Palladium-auf-Kohle-Katalysators hydriert; nach beendeter Wasserstoffaufnahme ist das Ausgangsmaterial gelöst. Der Katalysator wird abfiltriert und mit Methanol gewaschen; das Filtrat wird auf 200 g eingeengt und bei 60° mit 2,8 ml Triethylamin (20 mMol) versetzt, wobei das Produkt sofort zu kristallisieren beginnt. Man engt auf 100 g ein, lässt einige Zeit bei etwa 4° stehen, filtriert die Kristalle ab und wäscht mit einem Gemisch aus Methanol und Wasser chloridfrei. Das so erhältliche H-Gln(Trt)-OH enthält 0,5 Mol Wasser und schmilzt bei 233° (unter Zersetzung).

### Beispiel 8: Fmoc-Gln(Trt)-OH

Eine Lösung von 4 g des Halbhydrates von H-Gln(Trt)-OH (10 mMol) in 20 ml Tetrahydrofuran und 10 ml einnormaler wässriger Natriumhydroxidlösung wird unter gutem Rühren mit 3,4 g 9-Fluorenylmethyl-N-succinimidyl-carbonat (10 mMol) versetzt und gleichzeitig 1,4 ml Triethylamin so zugetropft, dass der pH-Wert etwa 8,5 beträgt. Nach 20 Minuten wird im Eisbad gekühlt, mit Essigsäureethylester überschichtet und mit 25 ml einmolarer wässriger Kaliumhydrogensulfatlösung bis zu einem pH-Wert von 2-2,5 angesäuert. Die organische Phase wird mit Wasser neutral gewaschen, über Natriumsulfat getrocknet und unter vermindertem Druck auf 30 g eingeengt. Das Produkt wird durch portionenweise Zugabe von 35 ml Diisopropylether zum Kristallisieren gebracht. Nach längerem Stehen in der Kälte wird das kristalline Material abfiltriert und einmal in einem 1:4-Gemisch von Essigsäureethylester und Diisopropylether und dreimal mit Diisopropylether gewaschen. Das Fmoc-Gln(Trt)-OH enthält auch nach längerem Trocknen im Hochvakuum bei 60° noch 0,5 Mol Diisopropylether, Smp. ab 125° (langsames Schmelzen unter Abgabe von Diisopropylether).

### Beispiel 9: Fmoc-Gln(Trt)-OH

Ein Gemisch von 7,4 g Fmoc-Gln-OH (20 mMol) und 10,4 g Triphenylmethanol (40 mMol) in 300 ml Essigsäure wird 10 Minuten bei 100° gerührt. Nach Zugabe von 0,1 ml konz. Schwefelsäure wird noch weitere 15 Minuten bei 100° gerührt, wobei eine klare, gelbe Lösung entsteht. Diese wird unter vermindertem Druck bei 60° eingedampft, und der Rückstand noch 10 Minuten unter vermindertem Druck getrocknet. Der feste Rückstand wird in 60 ml Essigsäure bei 60° gelöst, während 20 Minuten bei 60° belassen, dann mit 2 ml Essigsäureanhydrid (20 mMol) versetzt und nach 1 Stunde bei 60° unter vermindertem Druck eingedampft. Der ölige Rückstand wird während 10 Minuten bei 60° getrocknet, dann in 100 ml Essigsäure gelöst und in 500 ml eiskaltes Wasser eingetropft. Der Niederschlag wird abfiltriert, gut mit Wasser gewaschen und in Essigsäureethylester gelöst; die wässrige Phase wird abgetrennt, und die organische Phase mit einer gesättigten wässrigen Natriumchloridlösung gewaschen. Nach dem Trocknen über Natriumsulfat wird die organische Lösung zur Trockne eingedampft, der Rückstand wird in 50 ml 1,2-Dichlorethan gelöst und auf 100 g Silikagel gegeben; eluiert wird mit 1,2-Dichlorethan und 1,2-Dichlorethan enthaltend 2 %, dann 4 % Methanol. Die das Fmoc-Gln(Trt)-OH enthaltenden Fraktionen werden unter vermindertem Druck eingedampft, und das Produkt wird aus einem Gemisch von Essigsäureethylester und Diisopropylether kristallisiert, Smp. ab 125°.

### Beispiel 10: H-Gln-Gln-Gln-Gln-Gln-Ser-Leu-Gly-OH

Das Peptid wird zweimal mittels der Merrifield-Festphasensynthese hergestellt, wobei bei der einen Synthese Fmoc-Gln-OH und bei der anderen Fmoc-Gln(Trt)-OH verwendet wird.

Ausgehend von 1 g sogenanntem Fmoc-Gly-p-Benzyloxybenzylester-polystyrolharz (1 % vernetzt) der Firma Novabiochem (Läufelfingen, Schweiz), worin die Carboxylgruppe des Glycins, dessen Aminogruppe durch 9-Fluorenyl-methoxycarbonyl (Fmoc) geschützt ist, mit 4-Methoxy-benzylalkohol verestert ist, wobei das Kohlenstoffatom der Methoxygruppe mit einem aromatischen Ring des zu 1 % mit Divinylbenzol vernetzten Polystyrolharzes, das gleichzeitig als Trägermaterial dient, verbunden ist, wird der Aufbau des Peptidmoleküls nach der Merrifield-Synthese durchgeführt. Dabei wird eine automatische Peptidsyntheseapparatur verwendet, die sich zur alternierenden Abspaltung der Aminoschutzgruppen, im vorliegenden Fall der Fmoc-Gruppe, und zur Ankupplung der N-Fmoc-geschützten Aminosäureverbindungen eignet. Im ersten Schritt wird dabei die Fmoc-Schutzgruppe im Fmoc-Gly-p-Benzyloxybenzylester-Polystyrolharz entfernt und Fmoc-Leu-OH mit dem so erhaltenen H-Gly-p-Benzyloxybenzylester-Polystyrolharz gekuppelt, und dann die weiteren N-Fmoc-Aminosäuren stufenweise in der Reihenfolge Fmoc-Ser(But)-OH, dann entweder Fmoc-Gln-OH, Fmoc-Gln-OH, Fmoc-Gln-OH, Fmoc-Gln-OH und Fmoc-Gln-OH, oder Fmoc-Gln(Trt)-OH, Fmoc-Gln(Trt)-OH, Fmoc-Gln(Trt)-OH, Fmoc-Gln(Trt)-OH und Fmoc-Gln(Trt)-OH an das jeweils erhaltene Peptid/Harz-Zwischenprodukt gekuppelt. Die einzelnen Stufen werden nach folgendem Schema durchgeführt, wobei jeweils etwa 10 ml der Waschflüssigkeiten verwendet und die einzelnen Operationen, falls nicht anders angegeben, bei Raumtemperatur durchgeführt werden und wobei das Reaktionsgemisch jeweils regelmässig geschüttelt wird:

Ausgehend von 1 g des oben beschriebenen Fmoc-Gly/Harz-Ausgangsmaterials (Beladung: 0,47 mMol/g) werden die folgenden, sich für jede Stufe jeweils repetierenden Verfahrensschritte durchgeführt:
1. einmaliges Waschen während 0,8 Minuten mit Isopropanol;
2. dreimaliges Waschen während je 0,5 Minuten mit unter vermindertem Druck entgastem Dimethylacetamid (Dimethylamin-frei);
3. fünfmaliges Behandeln während je 1,7 Minuten mit einem 1:4-Gemisch von Piperidin und Dimethylacetamid (Abspaltung der Fmoc-Schutzgruppe);
4. einmaliges Waschen während 0,8 Minuten mit Isopropanol;
5. dreimaliges Waschen während je 0,5 Minuten mit unter vermindertem Druck entgastem Dimethylacetamid (Dimethylamin-frei);
6. einmaliges Waschen während 0,8 Minuten mit Isopropanol;
7. dreimaliges Behandeln während je 1,7 Minuten mit einem 1:4-Gemisch von Piperidin und Dimethylacetamid (zur vollständigen Abspaltung der Fmoc-Schutzgruppe);
8. zweimaliges Waschen während je 0,5 Minuten mit unter vermindertem Druck entgastem Dimethylacetamid (Dimethylamin-frei);
9. einmaliges Waschen während 0,8 Minuten mit Isopropanol;
10. sechsmaliges Waschen während je 0,5 Minuten mit unter vermindertem Druck entgastem Dimethylacetamid (Dimethylamin-frei);
11. Zugabe des zwischenzeitlich bereitgestellten Kupplungsreagens (das wie folgt erhalten wird: Ein Gemisch von 1,4 mMol der jeweiligen Fmoc-L-Aminosäure, 1,4 mMol 1-Hydroxy-1H-benztriazol und 1,55 mMol Diisopropylcarbodiimid in 4 ml Dimethylacetamid (Dimethylamin-frei) wird während etwa 30 Minuten bei Raumtemperatur belassen und dann verwendet). Die Kupplungsreaktion selber dauert 60 Minuten bei Raumtemperatur;
12. einmalige Acetylierung während 5 Minuten mit einem 1:1:8-Gemisch von Acetanhydrid, Pyridin und Dimethylacetamid (Dimethylamin-frei);
13. dreimaliges Waschen während je 0,5 Minuten mit unter vermindertem Druck entgastem Dimethylacetamid (Dimethylamin-frei);
14. einmaliges Waschen während 0,8 Minuten mit Isopropanol und
15. zweimaliges Waschen während 0,5 Minuten mit unter vermindertem Druck entgastem Dimethylacetamid (Dimethylamin-frei).

Nach Ankupplung der letzten Aminosäure werden nochmals die obigen Behandlungsschritte 1-10 zwecks Abspaltung der Fmoc-Gruppe ausgeführt.

Die Ausbeuten für jeden der letzten fünf Kupplungsschritte mit Fmoc-Gln-OH bzw. Fmoc-Gln(Trt)-OH werden anhand des mit der jeweiligen Abspaltung der Fmoc-Gruppe als Nebenprodukt gebildeten 1-(9-Fluorenylmethyl)-piperidins bestimmt. Hierzu werden jeweils die vereinigten Filtrate der Schritte 3-10 auf ein bestimmtes Volumen verdünnt und photometrisch im UV (λₘₐₓ= 299,8 nm, ε = 7800) ausgewertet. Die Ausbeuten betragen für die Serie mit Fmoc-Gln-OH 99 %, 97 %, 98 %, 49 % und 72 %, für diejenige mit Fmoc-Gln(Trt)-OH 100 %, 100 %, 99 %, 99 % und 100 %.

Das Peptid wird durch Behandeln mit etwa 10 ml eines (90:5:5)-Gemisches von Trifluoressigsäure, Wasser und 1,2-Ethandithiol während 8 Minuten von Harz abgespalten; das Reaktionsgemisch wird filtriert, das Filtrat unter vermindertem Druck eingeengt und das Peptid mit einem 1:1-Gemisch von Diisopropylether und Hexan ausgefällt und getrocknet. Durch die obengenannte Behandlung mit Trifluoressigsäure-Wasser-1,2-Ethandithiol werden teilweise bereits auch die Tritylgruppen (sofern vorhanden) und die tert.-Butylgruppe abgespalten. Diese Abspaltung wird durch 30minütiges Erwärmen der Peptidfällung auf 30°C in Trifluoressigsäure-Wasser-1,2-Ethandithiol (90:5:5) vervollständigt. Die erhaltenen Rohprodukte aus den Synthesen mit und ohne Trityl-Schutzgruppen werden im HPLC untersucht (Säule: Nucleosil 5C18, 250 x 4,6 mm; Laufmittel: A = 0,1 % Trifluoressigsäure in Wasser; B = 0,1 % Trifluoressigsäure in Acetonitril. Gradient: 0 bis 40 % B in 30 Minuten; Fluss: 1 ml/Minute. Detektion: 215 nm).

Das gewünschte Titelprodukt weist eine Retentionszeit von 17,98 Minuten auf. Das HPLC-Profil des Rohprodukts aus der Synthese mit Trityl-Schutz der Glutaminreste weist neben dem Titelprodukt (92 %) ein Signal (8 %) bei einer Retentionszeit von 18,65 Minuten auf. Das HPLC-Profil des Rohprodukts aus der Synthese ohne Trityl-Schutz der Glutaminreste weist neben dem Titelprodukt (nur 30 %) ein Signal (70 %) mit einer Retentionszeit von 18,38 Minuten auf.

### Beispiel 11: H-Arg-Arg-Ser-Asn-Gln-Val-Ser-Gln-Asn-Tyr-Pro-Ile-Val-Gln-Asn-Ile-Gln-Gly-Arg-Arg-OH

Das Titelpeptid wird zweimal nach dem im Beispiel 10 beschriebenen Syntheseschema hergestellt, erstens mit ungeschützten Fmoc-Asn-OH bzw. Fmoc-Gln-OH und zweitens mit Fmoc-Asn(Trt)-OH bzw. Fmoc-Gln(Trt)-OH. Ausgehend von Fmoc-Arg(Pmc)-Harz (Novabiochem) werden nacheinander folgende Aminosäuren ankondensiert: Fmoc-Arg(Pmc), Fmoc-Gly, Fmoc-Gln bzw. Fmoc-Gln(Trt), Fmoc-Ile, Fmoc-Asn bzw. Fmoc-Asn(Trt), Fmoc-Gln bzw. Fmoc-Gln(Trt), Fmoc-Val, Fmoc-Ile, Fmoc-Pro, Fmoc-Tyr(But), Fmoc-Asn bzw. Fmoc-Asn(Trt), Fmoc-Gln bzw. Fmoc-Gln(Trt), Fmoc-Ser(But), Fmoc-Val, Fmoc-Gln bzw. Fmoc-Gln(Trt), Fmoc-Asn bzw. Fmoc-Asn(Trt), Fmoc-Ser(But), Fmoc-Arg(Pmc) und Fmoc-Arg(Pmc).

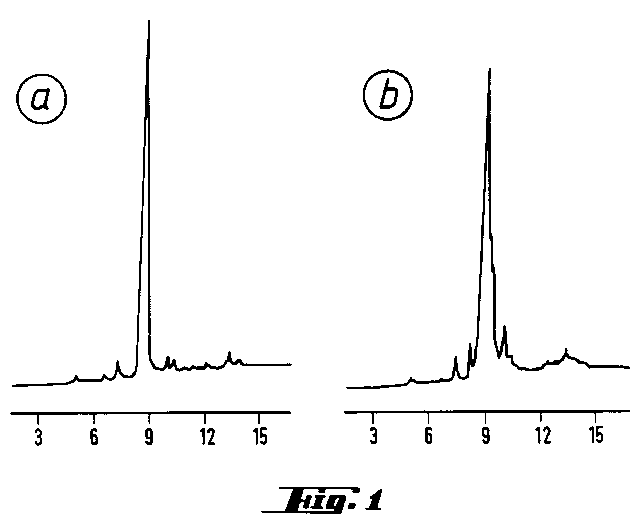

Das Peptid wird nach Abspaltung der N-terminalen Fmoc-Gruppe während 8 Minuten mit Trifluoressigsäure-H₂O-1,2-Ethandithiol (90:5:5) vom Harz abgespalten und anschliessend mit Trifluoressigsäure-H₂O-1,2-Ethandithiol-4-Methylmercaptophenol (90:5:3:2) während 90 Minuten bei 30° von den restlichen Schutzgruppen befreit. Das Rohprodukt aus der Synthese a (mit Trityl-Schutz) liefert im FAB-MS ein (M+H)⁺-Signal bei 2414,1 (berechnetes Molekulargewicht: 2413,7). Die HPLC-Profile (Säule: Nucleosil 7C18, 125 x 4,6 mm; Laufmittel: A = 0,1 % Trifluoressigsäure in Wasser; B = 0,1 % Trifluoressigsäure in Acetonitril. Gradient: 0 bis 90 % B in 30 Minuten, 1,5 ml/Minute. Detektion: 215 nm) der Rohprodukte von beiden Synthesen (a: mit Trityl-Schutz bei Asn und Gln; b: ohne Trityl-Schutz bei Asn und Gln) sind in Figur 1 abgebildet. Darin gibt die Abszissenskala die Retentionszeit in Minuten an.

### Beispiel 12: Fmoc-Lys-Gln-His-Asn-Pro-Lys-Tyr-Gln-Trp-Asn-OH

Zu einer Suspension von 10 g sogenanntem 4-Benzyloxybenzylalkoholpolystyrol (0,78 mMol OH/g; 1 % mit Divinylbenzol quervernetzt; die Phenylgruppen des Polystyrols sind in 4-Stellung durch 4-Hydroxymethylphenyloxymethyl substituiert; Novabiochem), 9,3 g (15,6 mMol) Fmoc-Asn(Trt)-OH und 3,1 ml (38,5 mMol) Pyridin in 60 ml Dimethylacetamid werden 3,35 ml (23,4 mMol) 2,6-Dichlor-benzoylchlorid (Fluka) in 4 Portionen innerhalb von 5 Stunden zugegeben und 24 Stunden gerührt. Das Harz wird abfiltriert und sehr gut mit Methanol und 1,2-Dichlor-ethan ausgewaschen. Das Harz weist eine Beladung von 0,48 mMol/g auf (photometrische Fmoc Bestimmung). Die restlichen OH-Gruppen werden vor Beginn der Peptidsynthese 2 Stunden lang mit Acetanhydrid-Pyridin-Dimethylacetamid 1:1:8 acetyliert. In analoger Weise (Ersatz von Fmoc-Asn(Trt)-OH durch Fmoc-Asn(Tmob)-OH) stellt man ein Fmoc-Asn(Tmob)-Harz her. Dieses weist eine Beladung von 0,44 mMol/g auf. 0,5 g Fmoc-Asn(Trt)-Harz (0,24 mMol Asn) bzw. 0,5 g Fmoc-Asn(Tmob)-Harz (0,22 mMol Asn) werden nach dem im Beispiel 10 beschriebenen Syntheseschema nacheinander mit folgenden Aminosäuren gekuppelt: Fmoc-Trp, Fmoc-Gln(Trt) bzw. Fmoc-Gln(Tmob), Fmoc-Tyr(But), Fmoc-Lys(Boc), Fmoc-Pro, Fmoc-Asn(Trt) bzw. Fmoc-Asn(Tmob), Fmoc-His(Trt), Fmoc-Gln(Trt) bzw. Fmoc-Gln(Tmob), und Fmoc-Lys(Boc).

Die erhaltenen Peptid-Harze werden 15 Minuten mit Trifluoressigsäure-Dimethylsulfid-1,2-Ethandithiol (77:20:3) geschüttelt, um das Peptid vom Harz abzuspalten. Die restlichen Schutzgruppen werden dann mit Trifluoressigsäure-Wasser-1,2-Ethandithiol (90:5:5) während 40 Minuten bei Raumtemperatur abgespalten.

Die erhaltenen Rohprodukte aus den Synthesen mit Trityl-Schutz an Asn und Gln (= Rohprodukt A) einerseits und mit Tmob-Schutz an Asn und Gln (= Rohprodukt B) andererseits werden im HPLC untersucht (Säule: Nucleosil 5C18, 250 x 4,6 mm; Laufmittel: A = 0,1 % Trifluoressigsäure in Wasser; B = 0,1 % Trifluoressigsäure in Acetonitril. Gradient: 0 bis 90 % B in 30 Minuten, 1 ml/Minute. Detektion: 215 nm). Das gewünschte Titelprodukt weist eine Retentionszeit von 29,8 Minuten auf. Das HPLC-Profil des Rohprodukts aus der Synthese mit Trityl-Schutz (Rohprodukt A) weist neben dem Titelprodukt (95 %, (M+H)⁺ im FAB-MS = 1565,4; berechnetes Molekulargewicht: 1564,7) ein Signal (5 %) bei einer Retentionszeit von 30,9 Minuten für

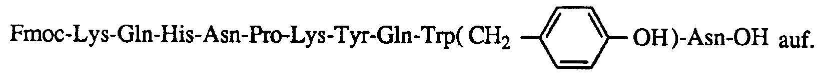

Das HPLC-Profil von Rohprodukt B (mit Tmob-Schutz) weist neben dem Signal für das Titelprodukt (nur etwa 8 %) und dem obengenannten Signal (ca. 3 %) mit der Retentionszeit von 30,9 Minuten ein Signal (89 %) mit einer Retentionszeit von 33,7 Minuten für 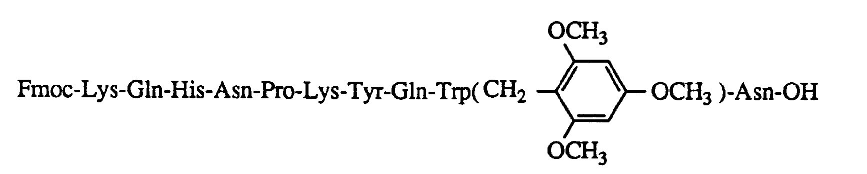
[(M+H)⁺ im FAB-MS = 1745,6; berechnetes Molekulargewicht: 1744,9] und weitere, sehr kleine Signale auf.

### Beispiel 13: Fmoc-Asn(Trt)-OTcp

Eine Lösung von 1,5 g (2,51 mMol) Fmoc-Asn(Trt)-OH und 0,54 g (2,75 mMol) 2,4,5-Trichlor-phenol in 10 ml Tetrahydrofuran wird auf 0° gekühlt. Man gibt 0,57 g (2,75 mMol) Dicyclohexyl-carbodiimid in fester Form zu und rührt während 30 Minuten bei 0°, 2 Stunden bei Raumtemperatur, nochmals 30 Minuten bei 0° und filtriert dann den gebildeten Dicyclohexylharnstoff ab. Aus dem Filtrat wird Fmoc-Asn(Trt)-OTcp durch Zugabe von 60 ml Hexan auskristallisiert; Smp. 133-135°, [α]_{D}²⁰ = +51,1° (THF).

### Beispiel 14: Fmoc-Gln(Trt)-OTcp

10 g (15,1 mMol) Fmoc-Gln(Trt)-OH x 0,5 Diisopropylether und 3,26 g (16,6 mMol) 2,4,5-Trichlor-phenol werden in 100 ml Tetrahydrofuran gelöst und auf 0° abgekühlt. Nach Zugabe von 3,41 g (16,6 mMol) Dicyclohexylcarbodiimid in fester Form wird 1 Stunde bei 0°, 2 Stunden bei Raumtemperatur und nochmals 30 Minuten bei 0° gerührt. Der auskristallisierte Dicyclohexylharnstoff wird abfiltriert und aus dem Filtrat Fmoc-Gln(Trt)-OTcp durch Zugabe von 750 ml Petrolether als Gallerte ausgefällt, abfiltriert, getrocknet und aus Methylenchlorid-Hexan kristallisiert; Smp. = 166°, [α]_{D}²⁰ = -16,1° (THF).

### Beispiel 15: Fmoc-Asn(Trt)-OPfp

1,5 g (2,51 mMol) Fmoc-Asn(Trt)-OH werden in 15 ml Tetrahydrofuran gelöst. Man gibt 0,51 g (2,76 mMol) Pentafluor-phenol zu, kühlt die Lösung auf 0° und fügt dann noch 0,57 g (2,76 mMol) Dicyclohexyl-carbodiimid in fester Form zu. Es wird während 30 Minuten bei 0°, 2 Stunden bei Raumtemperatur und nochmals 30 Minuten bei 0° gerührt. Der auskristallisierte Dicyclohexylharnstoff wird abfiltriert, das Filtrat zur Trockne eingeengt und mit 40 ml Diisopropylether verrieben. Der pulvrige Rückstand wird abfiltriert, getrocknet und aus Methylenchlorid-Petrolether umkristallisiert. Man erhält Fmoc-Asn(Trt)-OPfp; Smp. 154-156°, [α]_{D}²⁰ = -7,8° (THF).

### Beispiel 16: Fmoc-Gln(Trt)-OPfp

1,5 g (2,27 mMol) Fmoc-Gln(Trt)-OH x 0,5 Diisopropylether und 0,46 g (2,5 mMol) Pentafluor-phenol werden in 35 ml Essigsäureethylester gelöst, auf 0° gekühlt und unter Rühren mit 0,52 g (2,5 mMol) Dicyclohexylcarbodiimid versetzt. Man rührt während 1 Stunde bei 0° und 3 Stunden bei Raumtemperatur, filtriert das unlösliche Material ab und wäscht mit Tetrahydrofuran. Das Filtrat wird zur Trockne eingeengt und der Rückstand in 30 ml Tetrahydrofuran gelöst. Durch langsame Zugabe von Petrolether unter Rühren wird Fmoc-Gln(Trt)-OPfp kristallisiert; Smp. 185-187°, [α]_{D}²⁰ = -16,4° (THF).

### Beispiel 17: Fmoc-Asn(Trt)-ODhbt

1,5 g (2,51 mMol) Fmoc-Asn(Trt)-OH werden in 10 ml Tetrahydrofuran gelöst und mit einer Lösung von 0,45 g (2,76 mMol) 3,4-Dihydro-3-hydroxy-4-oxo-1,2,3-benzotriazin in 5 ml Dimethylformamid vermischt. Man kühlt auf 0°, gibt 0,57 g (2,76 mMol) Dicyclohexylcarbodiimid in fester Form zu und rührt während 1 Stunde bei 0°, 3 Stunden bei Raumtemperatur und dann nochmals 1 Stunde bei 0°. Der ausgeschiedene Dicyclohexylharnstoff wird abfiltriert, das Filtrat zur Trockne eingeengt, der Rückstand mit 30 ml Diisopropylether verrieben, abfiltriert und getrocknet. Das Rohprodukt wird durch Umfällen aus Methylenchlorid-Hexan gereinigt. Man erhält Fmoc-Asn(Trt)-ODhbt als amorphes Pulver; Smp. ca. 140°, [α]_{D}²⁰ = -40,8° (THF).

### Beispiel 18: Fmoc-Gln(Trt)-ODhbt

1 g (1,51 mMol) Fmoc-Gln(Trt)-OH x 0,5 Diisopropylether werden in 10 ml Essigsäureethylester gelöst. Man gibt 0,27 g (1,66 mMol) 3,4-Dihydro-3-hydroxy-4-oxo-1,2,3-benzotriazin in fein pulverisierter Form zu, und versetzt die Suspension nach Abkühlen auf 0° noch mit 0,34 g (1,66 mMol) Dicyclohexyl-carbodiimid in fester Form. Nach einstündigem Rühren bei 0° gibt man nochmals 10 ml Essigsäureethylester zu, rührt während 3 Stunden bei Raumtemperatur, filtriert den Dicyclohexylharnstoff ab und wäscht mit Essigsäureethylester. Das Filtrat wird auf ca. 10 ml konzentriert und daraus Fmoc-Gln(Trt)-ODhbt durch Zugabe von 50 ml Petrolether auskristallisiert; Smp. 130-133°, [α]_{D}²⁰ = -53,7° (THF).

### Beispiel 19: H-Asn(Trt)-OH · 0,5 H₂O

20 g (0,1515 Mol) H-Asn-OH und 78,8 g (0,303 Mol) Trt-OH werden in 455 ml Eisessig suspendiert. Man gibt 9,28 ml (0,174 Mol) konzentrierte H₂SO₄ zu, gefolgt von 28,6 ml (0,303 Mol) Acetanhydrid und rührt während 75 Minuten bei 60°. Nach ca. 40-50 Minuten ist alles gelöst. Das Reaktionsprodukt wird durch langsames Eintragen in 910 ml eisgekühltes H₂O ausgefällt. Anschliessend wird der pH-Wert mit ca. 850 ml 10 N NaOH auf ca. 6 gestellt. Nach ca. 1 Stunde Rühren bei 0° wird abfiltriert, mehrmals mit H₂O und dann mit Toluol gewaschen, bis kein Trt-OH mehr im Filtrat enthalten ist. Das erhaltene Titelprodukt zersetzt sich langsam ab 220°.

### Beispiel 20: Boc-Asn(Trt)-OH

3,83 g (10 mMol) H-Asn(Trt)-OH · 0,5 H₂O und 1,37 ml (10 mMol) Triethylamin werden in 38 ml 90%igem Tetrahydrofuran gelöst. Man gibt 2,2 ml (10 mMol) Di-tert.butyldicarbonat zu, gefolgt von weiteren 1,37 ml Triethylamin. Man lässt während 2¹/₂ Stunden bei Raumtemperatur reagieren und extrahiert dann mit 250 ml Essigsäureethylester und 125 ml wässriger einmolarer KHSO₄-Lösung. Die organische Phase wird neutral gewaschen, über Na₂SO₄ getrocknet und zur Trockne eingeengt. Der Rückstand wird aus Methanol-Essigsäureethylester-Hexan kristallisiert; Smp. des erhaltenen Titelprodukts: 201-203°, [α]_{D}²⁰ = -17,7±1° (THF).

### Beispiel 21: Boc-Gln(Trt)-OH

3,98 g (10 mMol) H-Gln(Trt)-OH · 0,5 H₂O und 1,38 ml (10 mMol) Triethylamin werden in 60 ml 90%igem Tetrahydrofuran suspendiert, dann mit 2,28 ml (10,2 mMol) (Boc)₂O und weiteren 1,38 ml Triethylamin versetzt und während 2 Stunden bei Raumtemperatur gerührt. Nach ca. 30 Minuten ist alles klar in Lösung gegangen. Man verfährt weiter analog Beispiel 20, mit dem Unterschied, dass der Rückstand aus Methanol-CH₂Cl₂-Petrolether kristallisiert wird; Smp. des erhaltenen Titelprodukts: ca. 112°, [α]_{D}²⁰ = -2±1° (THF).

## Patentansprüche (Patentansprüche für folgende(n) Vertragsstaat(en): AT, BE, CH, DE, DK, FR, GB, GR, IT, LI, LU, NL, SE)

1. Eine Verbindung der Formel I, 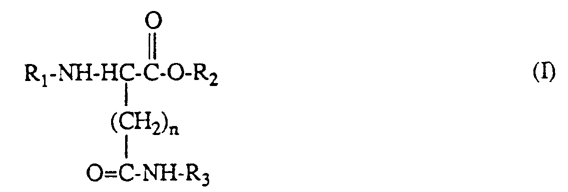 worin n für 1 oder 2 steht, R₁ Wasserstoff oder eine Aminoschutzgruppe, R₂ Wasserstoff oder eine Carboxylschutzgruppe und R₃ Triphenylmethyl, 4-Monomethoxy-trityl oder 4,4'-Dimethoxy-trityl bedeuten, ein Salz einer solchen Verbindung worin R₂ Wasserstoff ist mit einer salzbildenden Gruppe oder ein reaktionsfähiges Carbonsäurederivat einer solchen Verbindung der Formel I, worin R₂ für Wasserstoff steht, und ausserdem R₁ eine Aminoschutzgruppe ist.

2. Eine Verbindung nach Anspruch 1 der Formel I, worin R₁ eine Aminoschutzgruppe, R₂ Wasserstoff und R₃ Triphenylmethyl, 4-Monomethoxy-trityl oder 4,4'-Dimethoxy-trityl bedeuten, oder ein Metall- oder Ammoniumsalz davon.

3. Eine Verbindung nach Anspruch 1 der Formel I, worin R₁ Benzyloxycarbonyl, 9-Fluorenyl-methoxycarbonyl, Allyloxycarbonyl oder tert.-Butyloxycarbonyl, R₂ Wasserstoff und R₃ Triphenylmethyl bedeuten, oder ein Metall- oder Ammoniumsalz davon.

4. Eine Verbindung nach einem der Ansprüche 1 -3 der Formel I, worin R₁ 9-Fluorenylmethoxycarbonyl bedeutet, oder ein Metall- oder Ammoniumsalz einer solchen Verbindung, worin R₂ für Wasserstoff steht.

5. Benzyloxycarbonyl-Asn(triphenylmethyl)-OH oder ein Metall- oder Ammoniumsalz davon nach Anspruch 1.

6. 9-Fluorenyl-methoxycarbonyl-Asn(triphenylmethyl)-OH oder ein Metall- oder Ammoniumsalz davon nach Anspruch 1.

7. H-Asn(triphenylmethyl)-OH oder ein Salz davon nach Anspruch 1.

8. Tert.-butyloxycarbonyl-Asn(triphenylmethyl)-OH oder ein Salz davon nach Anspruch 1.

9. Benzyloxycarbonyl-Gln(triphenylmethyl)-OH oder ein Metall- oder Ammoniumsalz davon nach Anspruch 1.

10. 9-Fluorenyl-methoxycarbonyl-Gln(triphenylmethyl)-OH oder ein Metall- oder Ammoniumsalz davon nach Anspruch 1.

11. H-Gln(triphenylmethyl)-OH oder ein Salz davon nach Anspruch 1.

12. Tert.-butyloxycarbonyl-Gln(triphenylmethyl)-OH oder ein Salz davon nach Anspruch 1.

13. Ein reaktionsfähiges Carbonsäurederivat nach Anspruch 1 einer der in den Ansprüchen 2-6 sowie 8-11 und 12, beanspruchten Verbindungen.

14. Ein reaktionsfähiges Carbonsäurederivat nach Anspruch 13 in Form eines 2,4,5-Trichlor-phenylesters, Pentafluor-phenylesters, eines Säurechlorids, eines symmetrischen Anhydrids, eines Esters mit ¹-Hydroxybenztriazol oder eines Esters mit 3,4-Dihydro-3-hydroxy-4-oxo-1,2,3-benzomazin oder eines Esters mit N-Hydroxy-5-norbornen-2,3-dicarboximid.

15. 9-Fluorenyl-methoxycarbonyl-Asn(triphenylmethyl)-O-2,4,5-trichlorphenyl nach Anspruch 1.

16. 9-Fluorenyl-methoxycarbonyl-Gln(triphenylmethyl)-O-2,4,5-trichlorphenyl nach Anspruch 1.

17. 9-Fluorenyl-methoxycarbonyl-Asn(triphenylmethyl)-O-pentafluor-phenyl nach Anspruch 1.

18. 9-Fluorenyl-methoxycarbonyl-Gln(triphenylmethyl)-O-pentafluor-phenyl nach Anspruch 1.

19. 9-Fluorenyl-methoxycarbonyl-Asn(triphenylmethyl)-O-3,4-dihydro-3-hydroxy-4-oxo - 1,2,3-benzotriazinyl nach Anspruch 1.

20. 9-Fluorenyl-methoxycarbonyl-Gln(triphenylmethyl)-O-3,4-dihydro-3-hydroxy-4-oxo - 1,2,3-benzotriazinyl nach Anspruch 1.

21. Eine Substanz, die mindestens einen bivalenten Rest der Formel VII, 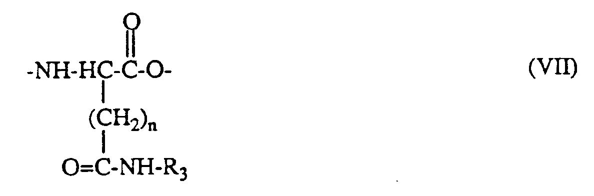 worin n für 1 oder 2 steht und R₃ Triphenylmethyl, 4-Monomethoxy-trityl oder 4,4'-Dimethoxy-trityl bedeutet, enthält.

22. Verfahren zur Herstellung einer Verbindung der Formel I, 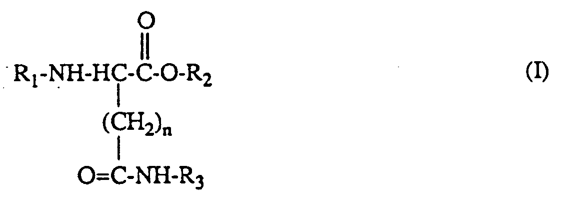 worin n für 1 oder 2 steht, R₁ Wasserstoff oder eine Aminoschutzgruppe, R₂ Wasserstoff oder eine Carboxylschutzgruppe und R₃ Triphenylmethyl, 4-Monomethoxy-trityl oder 4,4'-Dimethoxy-trityl bedeuten, eines Salzes einer solchen Verbindung worin R₂ Wasserstoff ist mit einer salzbildenden Gruppe oder eines reaktionsfähigen Carbonsäurederivats einer solchen Verbindung, worin R₂ für Wasserstoff steht und ausserdem R₁ eine Aminoschutzgruppe ist, dadurch gekennzeichnet, dass man
a) eine Verbindung der Formel II, 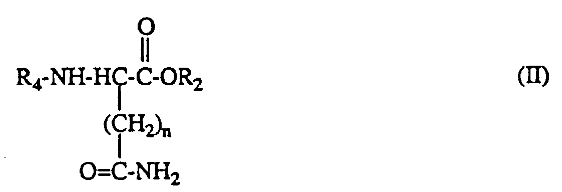 worin R₄ für Wasserstoff oder eine unter den Reaktionsbedingungen stabile Aminoschutzgruppe steht und n und R₂ die obengenannten Bedeutungen haben, mit einer Verbindung der Formel III,
R₃-OH (III)
worin R₃ die obengenannte Bedeutung hat, umsetzt und aus dem erhaltenen Produkt diejenigen Schutzgruppen abspaltet, die nicht Bestandteil des gewünschten Endprodukts der Formel I sind, oder
b) zur Herstellung einer Verbindung der Formel I, worin R₁ für eine Aminoschutzgruppe steht, in eine Verbindung der Formel IV, 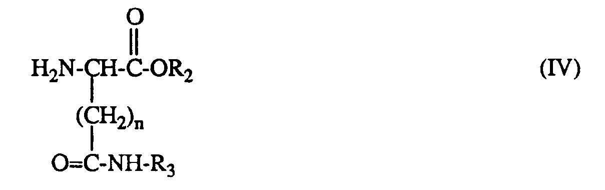 worin n, R₂ und R₃ die obengenannten Bedeutungen haben, eine Aminoschutzgruppe einführt, oder
c) eine Verbindung der Formel V, 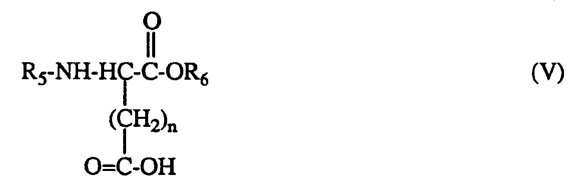 worin n die obengenannte Bedeutung hat, R₅ eine unter den Reaktionsbedingungen stabile Aminoschutzgruppe bedeutet und R₆ für eine unter den Reaktionsbedingungen stabile Carboxylschutzgruppe steht, mit einer Verbindung der Formel VI,
R₃-NH₂ (VI)
worin R₃ die obengenannte Bedeutung hat, umsetzt und aus dem erhaltenen Produkt diejenigen Schutzgruppen abspaltet, die nicht Bestandteil des gewünschten Endprodukts der Formel I sind,
und, wenn erwünscht, nach Durchführung eines der Verfahren a), b) oder c) eine erhaltene Verbindung der Formel I mit einer salzbildenden Gruppe in ihr Salz überführt oder ein erhaltenes Salz in die freie Verbindung überführt oder eines erhaltene Verbindung der Formel I, worin R₂ Wasserstoff und R₁ eine Aminoschutzgruppe bedeutet, in ein reaktionsfähiges Carbonsäurederivat überführt.

23. Verfahren nach Anspruch 22, dadurch gekennzeichnet, dass man eine Verbindung der Formel II, worin R₄ für Benzyloxycarbonyl oder 9-Fluorenyl-methoxycarbonyl steht, in einem geeigneten organischen Lösungsmittel in Gegenwart katalytischer Mengen einer wasserfreien starken Lewis-Säure und in Gegenwart eines wasserentziehenden Mittels zwischen +20°C und +70°C umsetzt.

24. Verwendung einer Verbindung der Formel I, worin R₂ für Wasserstoff steht, oder eines reaktionsfähigen Carbonsäurederivats davon nach einem der Ansprüche 1-20 zur Synthese von Peptiden an fester Phase.

## Patentansprüche (Patentansprüche für folgende(n) Vertragsstaat(en): ES)

1. Verfahren zur Herstellung einer Verbindung der Formel I, 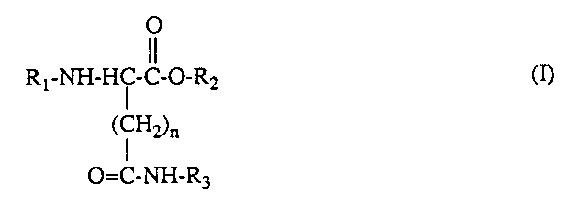 worin n für 1 oder 2 steht, R₁ Wasserstoff oder eine Aminoschutzgruppe, R₂ Wasserstoff oder eine Carboxylschutzgruppe und R₃ Triphenylmethyl, 4-Monomethoxy-trityl oder 4,4'-Dimethoxy-trityl bedeuten, eines Salzes einer solchen Verbindung worin R₂ Wasserstoff ist, mit einer salzbildenden Gruppe oder eines reaktionsfähigen Carbonsäurederivats einer solchen Verbindung, worin R₂ für Wasserstoff steht und ausserdem R₁ eine Aminoschutzgruppe ist, dadurch gekennzeichnet, dass man
a) eine Verbindung der Formel II, 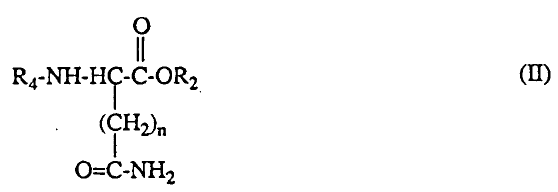 worin R₄ für Wasserstoff oder eine unter den Reaktionsbedingungen stabile Aminoschutzgruppe steht und n und R₂ die obengenannten Bedeutungen haben, mit einer Verbindung der Formel III,
R₃-OH (III)
worin R₃ die obengenannte Bedeutung hat, umsetzt und aus dem erhaltenen Produkt diejenigen Schutzgruppen abspaltet, die nicht Bestandteil des gewünschten Endprodukts der Formel I sind, oder
b) zur Herstellung einer Verbindung der Formel I, worin R₁ für eine Aminoschutzgruppe steht, in eine Verbindung der Formel IV, 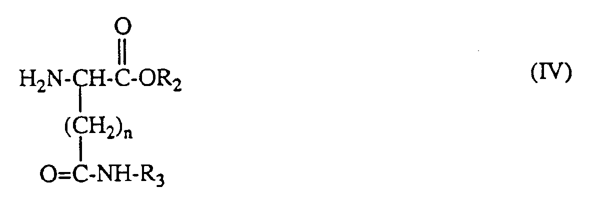 worin n, R₂ und R₃ die obengenannten Bedeutungen haben, eine Aminoschutzgruppe einführt, oder
c) eine Verbindung der Formel V, 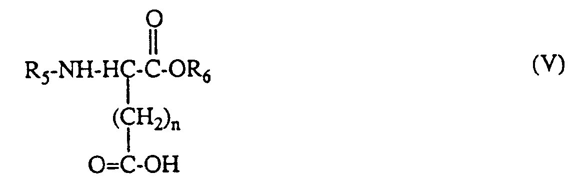 worin n die obengenannte Bedeutung hat, R₅ eine unter den Reaktionsbedingungen stabile Aminoschutzgruppe bedeutet und R₆ für eine unter den Reaktionsbedingungen stabile Carboxylschutzgruppe steht, mit einer Verbindung der Formel VI,
R₃-NH₂ (VI)
worin R₃ die obengenannte Bedeutung hat, umsetzt und aus dem erhaltenen Produkt diejenigen Schutzgruppen abspaltet, die nicht Bestandteil des gewünschten Endprodukts der Formel I sind,
und, wenn erwünscht, nach Durchführung eines der Verfahren a), b) oder c) eine erhaltene Verbindung der Formel I mit einer salzbildenden Gruppe in ihr Salz überführt oder ein erhaltenes Salz in die freie Verbindung überführt oder eine erhaltene Verbindung der Formel I, worin R₂ Wasserstoff und R₁ eine Aminoschutzgruppe bedeutet, in ein reaktionsfähiges Carbonsäurederivat überführt.

2. Verfahren nach Anspruch 1, dadurch gekennzeichnet, dass man eine Verbindung der Formel II, worin R₄ für Benzyloxycarbonyl oder 9-Fluorenyl-methoxycarbonyl steht, in einem geeigneten organischen Lösungsmittel in Gegenwart katalytischer Mengen einer wasserfreien starken Lewis-Säure und in Gegenwart eines wasserentziehenden Mittels zwischen +20°C und +70°C umsetzt.

3. Verfahren nach Anspruch 1 oder 2 zur Herstellung einer Verbindung der Formel I, worin R₁ eine Aminoschutzgruppe, R₂ Wasserstoff und R₃ Triphenylmethyl, 4-Monomethoxy-trityl oder 4,4'-Dimethoxy-trityl bedeuten, oder eines Metall- oder Ammoniumsalzes davon.

4. Verfahren nach Anspruch 1 oder 2 zur Herstellung einer Verbindung der Formel I, worin R₁ Benzyloxycarbonyl, 9-Fluorenyl-methoxycarbonyl, Allyloxycarbonyl oder tert.-Butyloxycarbonyl, R₂ Wasserstoff und R₃ Triphenylmethyl bedeuten, oder eines Metall- oder Ammoniumsalzes davon.

5. Verfahren nach Anspruch 1 oder 2 zur Herstellung einer Verbindung der Formel I, worin R₁ 9-Fluorenyl-methoxycarbonyl bedeutet, oder eines Metall- oder Ammoniumsalzes einer solchen Verbindung, worin R₂ für Wasserstoff steht.

6. Verfahren nach Anspruch 1 oder 2 zur Herstellung von Benzyloxycarbonyl-Asn(triphenylmethyl)-OH oder eines Metall- oder Ammoniumsalzes davon.

7. Verfahren nach Anspruch 1 oder 2 zur Herstellung von 9-Fluorenyl-methoxycarbonyl-Asn(triphenylmethyl)-OH oder eines Metall- oder Ammoniumsalzes davon.

8. Verfahren nach Anspruch 1 oder 2 zur Herstellung von H-Asn(triphenylmethyl)-OH oder eines Salzes davon.

9. Verfahren nach Anspruch 1 oder 2 zur Herstellung von Tert.-butyloxycarbonyl-Asn(triphenylmethyl)-OH oder eines Salzes davon

10. Verfahren nach Anspruch 1 oder 2 zur Herstellung von Benzyloxycarbonyl-Gln(triphenylmethyl)-OH oder eines Metall- oder Ammoniumsalzes davon.

11. Verfahren nach Anspruch 1 oder 2 zur Herstellung von 9-Fluorenyl-methoxycarbonyl-Gln(triphenylmethyl)-OH oder eines Metall- oder Ammoniumsalzes davon.

12. Verfahren nach Anspruch 1 oder 2 zur Herstellung von H-Gln(triphenylmethyl)-OH oder eines Salzes davon.

13. Verfahren nach Anspruch 1 oder 2 zur Herstellung von Tert.-butyloxycarbonyl-Gln(triphenylmethyl)-OH oder eines Salzes davon.

14. Verfahren nach Anspruch 1 oder 2 zur Herstellung eines reaktionsfähigen Carbonsäurederivates einer Verbindung der Formel I.

15. Verfahren nach Anspruch 1 oder 2 zur Herstellung eines reaktionsfähigen Carbonsäurederivates einer Verbindung der Formel I in Form eines 2,4,5-Trichlor-phenylesters, Pentafluor-phenylesters, eines Säurechlorids, eines symmetrischen Anhydrids, eines Esters mit 1-Hydroxybenztriazol oder eines Esters mit 3,4-Dihydro-3-hydroxy-4-oxo-1,2,3-benzotriazin oder eines Esters mit N-Hydroxy-5-norbornen-2,3-dicarboximid.

16. Verfahren nach Anspruch 1 oder 2 zur Herstellung von 9-Fluorenyl-methoxycarbonyl-Asn(triphenylmethyl)-O-2,4,5-trichlorphenyl.

17. Verfahren nach Anspruch 1 oder 2 zur Herstellung von 9-Fluorenyl-methoxycarbonyl-Gln(triphenylmethyl)-O-2,4,5-trichlorphenyl.

18. Verfahren nach Anspruch 1 oder 2 zur Herstellung von 9-Fluorenyl-methoxycarbonyl-Asn(triphenylmethyl)-O-pentafluor-phenyl.

19. Verfahren nach Anspruch 1 oder 2 zur Herstellung von 9-Fluorenyl-methoxycarbonyl-Gln(triphenylmethyl)-O-pentafluorphenyl.

20. Verfahren nach Anspruch 1 oder 2 zur Herstellung von 9-Fluorenyl-methoxycarbonyl-Asn(triphenylmethyl)-O-3,4-dihydro-3-hydroxy-4-oxo-1,2,3-benzotriazinyl.

21. Verfahren nach Anspruch 1 oder 2 zur Herstellung von 9-Fluorenyl-methoxycarbonyl-Gln(triphenylmethyl)-O-3,4-dihydro-3-hydroxy-4-oxo-1,2,3-benzotriazinyl.

22. Verfahren zur Herstellung einer Substanz, die mindestens einen bivalenten Rest der Formel VII 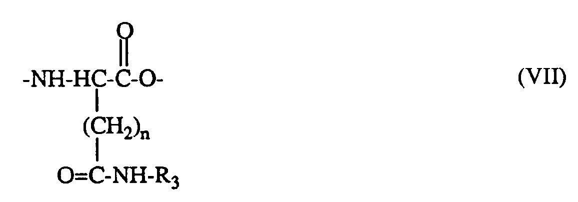 enthält, worin n für 1 oder 2 steht und R₃ Triphenylmethyl, 4-Monomethoxy-trityl oder 4,4'-Dimethoxy-trityl bedeutet, durch Peptidsynthese an fester Phase.

## Patentansprüche (Patentansprüche für folgende(n) Vertragsstaat(en): AT, BE, CH, DE, DK, FR, GB, GR, IT, LI, LU, NL, SE)

1. A compound of the formula I, 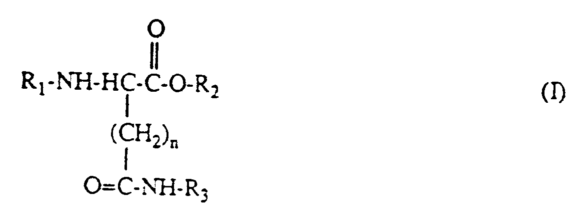 wherein n stands for 1 or 2, R₁ signifies hydrogen or an amino protective group, R₂ hydrogen or a carboxyl protective group and R₃ triphenylmethyl, 4-monomethoxytrityl or 4,4'-dimethoxy-trityl, a salt of such a compound, wherein R₂ is hydrogen, with a salt-forming group or a reactive carboxylic acid derivative of such a compound of the formula I, wherein R₂ stands for hydrogen and, moreover, R₁ is an amino protective group.

2. A compound according to claim 1 of the formula I, wherein R₁ signifies an amino protective group, R₂ hydrogen and R₃ triphenylmethyl, 4-monomethoxy -trityl or 4,4'-dimethoxy-trityl, or a metal salt or ammonium salt thereof.

3. A compound according to claim 1 of the formula I, wherein R₁ signifies benzyloxycarbonyl, 9-fluorenyl -methoxycarbonyl, allyloxycarbonyl or tert.-butyloxycarbonyl, R₂ hydrogen and R₃ triphenylmethyl, or a metal salt or ammonium salt thereof.

4. A compound according to one of claims 1-3 of the formula I, wherein R₁ signifies 9-fluorenyl-methoxycarbonyl, or a metal salt or ammonium salt of such a compound, wherein R₂ stands for hydrogen.

5. Benzyloxycarbonyl-Asn(triphenylmethyl)-OH or a metal salt or ammonium salt thereof according to claim 1.

6. 9-fluorenyl-methoxycarbonyl-Asn (triphenylmethyl)-OH or a metal salt or ammonium salt thereof according to claim 1.

7. H-Asn(triphenylmethyl)-OH or a salt thereof according to claim 1.

8. Tert.-butyloxycarbonyl-Asn (triphenylmethyl)-OH or a salt thereof according to claim 1.

9. Benzyloxycarbonyl-Gln(triphenylmethyl)-OH or a metal salt or ammonium salt thereof according to claim 1.

10. 9-fluorenyl-methoxycarbonyl-Gln (triphenylmethyl)-OH or a metal salt or ammonium salt thereof according to claim 1.

11. H-Gln(triphenylmethyl)-OH or a salt thereof according to claim 1.

12. Tert.-butyloxycarbonyl-Gln (triphenylmethyl)-OH or a salt thereof according to claim 1.

13. A reactive carboxylic acid derivative according to claim 1 of one of the compounds claimed in claims 2-6 and 8-11 and 12.

14. A reactive carboxylic acid derivative according to claim 13 in the form of a 2,4,5-trichloro-phenylester, pentafluoro-phenylester, an acid chloride, a symmetrical anhydride, an ester with '-hydroxybenztriazole or an ester with 3,4-dihydro-3-hydroxy -4-oxo-1,2,3-benzotriazine or an ester with N-hydroxy -5-norbornene-2,3-dicarboximide.

15. 9-fluorenyl-methoxycarbonyl-Asn (triphenylmethyl)-O-2,4,5-trichlorophenyl according to claim 1.

16. 9-fluorenyl-methoxycarbonyl-Gln (triphenylmethyl)-O-2,4,5-trichlorophenyl according to claim 1.

17. 9-fluorenyl-methoxycarbonyl-Asn (triphenylmethyl)-O-pentafluoro-phenyl according to claim 1.

18. 9-fluorenyl-methoxycarbonyl-Gln (triphenylmethyl)-O-pentafluoro-phenyl according to claim 1.

19. 9-fluorenyl-methoxycarbonyl-Asn (triphenylmethyl)-O-3,4-dihydro-3-hydroxy-4-oxo-1,2,3-benzotriazinyl according to claim 1.

20. 9-fluorenyl-methoxycarbonyl-Gln (triphenylmethyl)-O-3,4-dihydro-3-hydroxy-4-oxo-1,2,3-benzotriazinyl according to claim 1.

21. A substance which contains at least one bivalent residue of the formula VII, 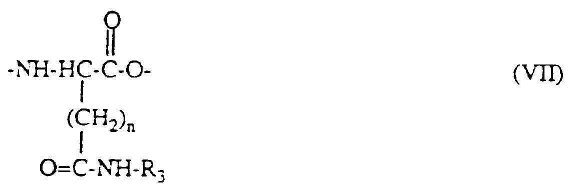 wherein n stands for 1 or 2 and R₃ signifies triphenylmethyl, 4-monomethoxy-trityl or 4,4'dimethoxy-trityl.

22. Process for the manufacture of a compound of the formula I, 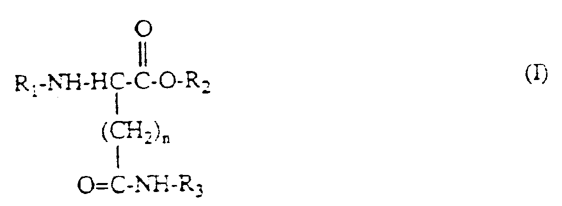 wherein n stands for 1 or 2, R₁ signifies hydrogen or an amino protective group, R₂ hydrogen or a carboxyl protective group and R₃ triphenylmethyl, 4-monomethoxytrityl or 4,4'-dimethoxy-trityl, a salt of such a compound, wherein R₂ is hydrogen, with a salt-forming group or a reactive carboxylic acid derivative of such a compound, wherein R₂ stands for hydrogen and,
moreover, R₁ is an amino protective group,
characterized in that
a) a compound of the formula II, 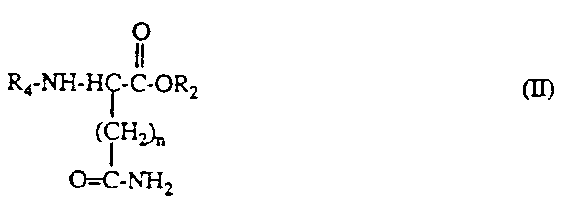 wherein R₄ stands for hydrogen or an amino protective group stable under the reaction conditions and n and R₂ have the aforementioned meanings, is caused to react with a compound of the formula III,
R₃-OH (III)
wherein R₃ has the aforementioned significance, and from the product obtained, those protective groups are cleaved which are not a constituent of the desired end product of the formula I, or
b) for the manufacture of a compound of the formula I, wherein R₁ stands for an amino protective group, an amino protective group is introduced into a compound of the formula IV, 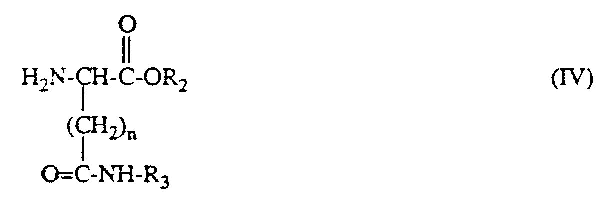 wherein n, R₂ and R₃ have the aforementioned meanings, or
c) a compound of the formula V, 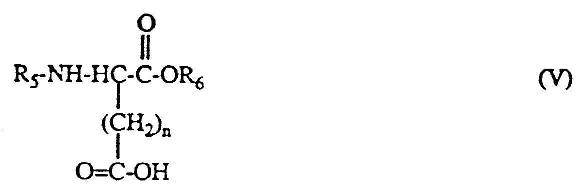 wherein n has the aforementioned significance, R₅ signifies an amino protective group stable under the reaction conditions and R₆ stands for a carboxyl protective group stable under the reaction conditions, is caused to react with a compound of the formula VI,
R₃-NH₂ (VI)
wherein R₃ has the aforementioned significance, and from the product obtained, those protective groups are cleaved which are not a constituent of the desired end product of the formula I,
and, if desired, after carrying out one of processes a), b) or c) converts an obtained compound of the formula I with a salt-forming group into its salt or converts an obtained salt into the free compound or converts an obtained compound of the formula I, wherein R₂ signifies hydrogen and R₁ an amino protective group, into a reactive carboxylic acid derivative.

23. Process according to claim 22,
characterized in that a compound of the formula II,
wherein R₄ stands for benzyloxycarbonyl or 9-fluorenyl-methoxycarbonyl, is caused to react in a suitable organic solvent in the presence of catalytic quantities of an anhydrous strong Lewis acid and in the presence of a dehydrating medium between +20°C and +70°C.

24. Use of a compound of the formula I,
wherein R₂ stands for hydrogen, or a reactive carboxylic acid derivative thereof according to one of claims 1-20 for the synthesis of peptides at a fixed stage.

## Claims (Claims for the following Contracting State(s): ES)

1. Process for the manufacture of a compound of the formula I, 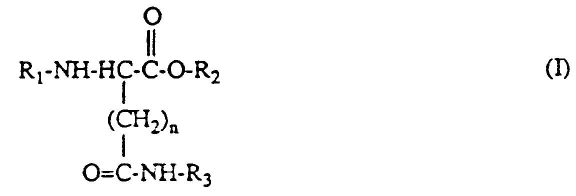 wherein n stands for 1 or 2, R₁ signifies hydrogen or an amino protective group, R₂ hydrogen or a carboxyl protective group and R₃ triphenylmethyl, 4-monomethoxytrityl or 4,4'-dimethoxy-trityl, a salt of such a compound, wherein R₂ is hydrogen, with a salt-forming group or a reactive carboxylic acid derivative of such a compound, wherein R₂ stands for hydrogen and,
moreover, R₁ is an amino protective group,
characterized in that
a) a compound of the formula II, 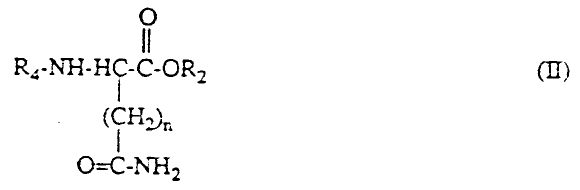 wherein R₄ stands for hydrogen or an amino protective group stable under the reaction conditions and n and R₂ have the aforementioned meanings, is caused to react with a compound of the formula III,
R₃ -OH (III)
wherein R₃ has the aforementioned significance, and from the product obtained, those protective groups are cleaved which are not a constituent of the desired end product of the formula I, or
b) for the manufacture of a compound of the formula I, wherein R₁ stands for an amino protective group, an amino protective group is introduced into a compound of the formula IV, 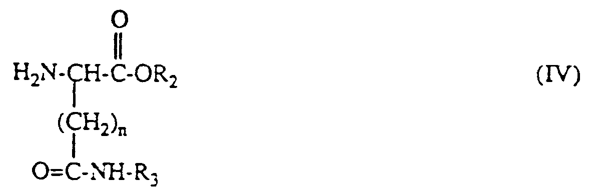 wherein n, R₂ and R₃ have the aforementioned meanings,
or
c) a compound of the formula V, 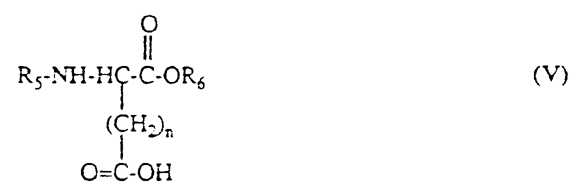 wherein n has the aforementioned significance, R₅ signifies an amino protective group stable under the reaction conditions and R₆ stands for a carboxyl protective group stable under the reaction conditions, is caused to react with a compound of the formula VI,
R₃-NH₂ (VI)
wherein R₃ has the aforementioned significance, and from the product obtained, those protective groups are cleaved which are not a constituent of the desired end product of the formula I,
and, if desired, after carrying out one of processes a), b) or c) converts an obtained compound of the formula I with a salt-forming group into its salt or converts an obtained salt into the free compound or converts an obtained compound of the formula I, wherein R₂ signifies hydrogen and R₁ an amino protective group, into a reactive carboxylic acid derivative.

2. Process according to claim 1,
characterized in that a compound of the formula II,
wherein R₄ stands for benzyloxycarbonyl or 9-fluorenylmethoxycarbonyl, is caused to react in a suitable organic solvent in the presence of catalytic quantities of an anhydrous strong Lewis acid and in the presence of a dehydrating medium between +20°C and +70°C.

3. Process according to claim 1 or 2 for the manufacture of a compound of the formula I, wherein R₁ signifies an amino protective group, R₂ hydrogen and R₃ triphenylmethyl, 4-monomethoxy-trityl or 4,4'dimethoxy-trityl, or a metal salt or ammonium salt thereof.

4. Process according to claim 1 or 2 for the manufacture of a compound of the formula I, wherein R₁ signifies benzyloxycarbonyl, 9-fluorenyl-methoxycarbonyl, allyloxycarbonyl or tert.-butyloxycarbonyl, R₂ hydrogen and R₃ triphenylmethyl, or a metal salt or ammonium salt thereof.

5. Process according to claim 1 or 2 for the manufacture of a compound of the formula I, wherein R₁ signifies 9-fluorenyl-methoxycarbonyl, or a metal salt or ammonium salt of such a compound, wherein R₂ stands for hydrogen.

6. Process according to claim 1 or 2 for the manufacture of benzyloxycarbonyl-Asn(triphenylmethyl)-OH or a metal salt or ammonium salt thereof.

7. Process according to claim 1 or 2 for the manufacture of 9-fluorenyl-methoxycarbonyl-Asn (triphenylmethyl)-OH or a metal salt or ammonium salt thereof.

8. Process according to claim 1 or 2 for the manufacture of H-Asn(triphenylmethyl)-OH or a salt thereof.

9. Process according to claim 1 or 2 for the manufacture of tert.-butyloxycarbonyl-Asn (triphenylmethyl)-OH or a salt thereof.

10. Process according to claim 1 or 2 for the manufacture of benzyloxycarbonyl-Gln (triphenylmethyl)-OH or a metal salt or ammonium salt thereof.

11. Process according to claim 1 or 2 for the manufacture of 9-fluorenyl-methoxycarbonyl-Gln (triphenylmethyl)-OH or a metal salt or ammonium salt thereof.

12. Process according to claim 1 or 2 for the manufacture of H-Gln(triphenylmethyl)-OH or a salt thereof.

13. Process according to claim 1 or 2 for the manufacture of tert.-butyloxycarbonyl-Gln (triphenylmethyl)-OH or a salt thereof.

14. Process according to claim 1 or 2 for the manufacture of a reactive carboxylic acid derivative of a compound of the formula I.

15. Process according to claim 1 or 2 for the manufacture of a reactive carboxylic acid derivative of a compound of the formula I in the form of a 2,4,5-trichloro-phenylester, pentafluoro-phenylester, an acid chloride, a symmetrical anhydride, an ester with 1-hydroxybenztriazole or an ester with 3,4-dihydro-3-hydroxy-4-oxo-1,2,3-benzotriazine or an ester with N-hydroxy-5-norbornene-2,3-dicarboximide.

16. Process according to claim 1 or 2 for the manufacture of 9-fluorenyl-methoxycarbonyl-Asn (triphenylmethyl)-O-2,4,5-trichlorophenyl.

17. Process according to claim 1 or 2 for the manufacture of 9-fluorenyl-methoxycarbonyl-Gln (triphenylmethyl)-O-2,4,5-trichlorophenyl.

18. Process according to claim 1 or 2 for the manufacture of 9-fluorenyl-methoxycarbonyl-Asn (triphenylmethyl)-O-pentafluoro-phenyl.

19. Process according to claim 1 or 2 for the manufacture of 9-fluorenyl-methoxycarbonyl-Gln(triphenylmethyl)-O-pentafluorophenyl.

20. Process according to claim 1 or 2 for the manufacture of 9-fluorenyl-methoxycarbonyl-Asn (triphenylmethyl)-O-3,4-dihydro-3-hydroxy-4-oxo-1,2,3-benzotriazinyl.

21. Process according to claim 1 or 2 for the manufacture of 9-fluorenyl-methoxycarbonyl-Gln (triphenylmethyl)-O-3,4-dihydro-3-hydroxy-4-oxo-1,2,3-benzotriazinyl.

22. Process for the manufacture of a substance which contains at least one bivalent residue of the formula VII, 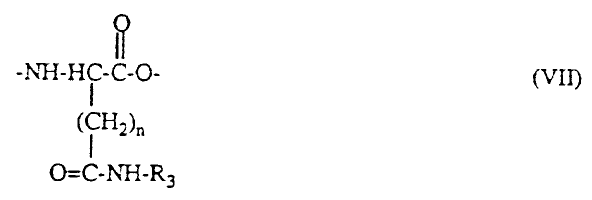 wherein n stands for 1 or 2 and R₃ signifies triphenylmethyl, 4-monomethoxy-trityl or 4,4'dimethoxy-trityl, through peptide synthesis at a fixed stage.

## Revendications (Revendications pour l'(les) Etat(s) contractant(s) suivant(s): AT, BE, CH, DE, DK, FR, GB, GR, IT, LI, LU, NL, SE)

1. Un composé de formule I, 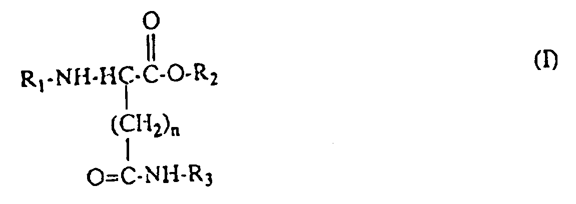 dans laquelle n désigne 1 ou 2, R₁ désigne l'hydrogène ou un groupe de protection de la fonction amino, R₂ désigne l'hydrogène ou un groupe de protection de la fonction carboxyle et R₃ désigne un radical triphénylméthyle, 4-monométhoxytrityle ou 4,4'-diméthoxytrityle, un sel d'un tel composé dans lequel R₂ désigne l'hydrogène avec un groupe formateur de sel ou un dérivé réactif d'acide carboxylique d'un tel composé de formule I, dans laquelle R₂ désigne l'hydrogène et, en outre, R₁ est un groupe de protection de la fonction amino.

2. Un composé selon la revendication 1 de formule I dans laquelle R₁ désigne un groupe de protection de la fonction amino, R₂ désigna l'hydrogène et R₃ désigne un radical triphénylméthyle, 4-monométhoxytrityle ou 4,4'diméthoxytrityle ou un sel de métal ou d'ammonium de celui-ci.

3. Un composé selon la revendication 1 de formule I dans laquelle R₁ désigne un radical benzyloxycarbonyle, 9-fluorénylméthoxycarbonyle, allyloxycarbonyle ou tert.-butyloxycarbonyle, R₂ désigne l'hydrogène et R₃ désigne un radical triphénylméthyle ou un sel de métal ou d'ammonium de celui-ci.

4. Un composé selon une des revendications 1 à 3 de formule I dans laquelle R₁ désigne un radical 9-fluorénylméthoxycarbonyle ou un sel de métal ou d'ammonium d'un tel composé dans lequel R₂ désigne l'hydrogéne.

5. Benzyloxycarbonyl-Asn(triphénylméthyl)-OH ou un de ses sels de métal ou d'ammonium selon la revendication 1.

6. 9-fluorénylméthoxycarbonyl-Asn(triphénylméthyl)-OH ou un de ses sels de métal ou d'ammonium selon la revendication 1.

7. H-Asn(triphénylméthyl)-OH ou un de ses sels selon la revendication 1.

8. Tert.-butyloxycarbonyl-Asn(triphénylméthyl)-OH ou un de ses sels selon la revendication 1.

9. Benzyloxycarbonyl-Gln(triphénylméthyl)-OH ou un de ses sels de métal ou d'ammonium selon la revendication 1.

10. 9-fluorénylméthoxycarbonyl-Gln(triphénylméthyl)-OH-ou un de ses sels de métal ou d'ammonium selon la revendication 1.

11. H-Gln(triphénylméthyl)-OH ou un de ses sels selon la revendication 1.

12. Tert.-butyloxycarbonyl-Gln(triphénylméthyl)-OH ou un de ses sels selon la revendication 1.

13. Un dérivé réactif d'acide carboxylique selon la revendication 1 d'un des composés revendiqués dans les revendications 2-6 ainsi que 8-11 et 12.

14. Un dérivé réactif d'acide carboxylique selon la revendication 13 sous la forme d'un 2,4,5-trichlorophénylester, d'un pentafluorophénylester, d'un chlorure d'acide, d'un anhydride symétrique, d'un ester avec le 1-hydroxybenzotriazole ou d'un ester avec la 3,4-dihydro-3-hydroxy-4 -oxo-1,2,3-benzotriazine ou d'un ester avec le N-hydroxy-5-norbornène-2,3-dicarboximide.

15. 9-fluorénylméthoxycarbonyl-Asn(triphénylméthyl)-O-2,4,5-trichlorophényle selon la revendication 1.

16. 9-fluorénylméthoxycarbonyl-Gln(triphénylméthyl)-O-2,4,5-trichlorophényle selon la revendication 1.

17. 9-fluorénylméthoxycarbonyl-Asn(triphénylméthyl)-O-pentafluorophényle selon la revendication 1.

18. 9-fluorénylméthoxycarbonyl-Gln(triphénylméthyl)-O-pentafluorophényle selon la revendication 1.

19. 9-fluorénylméthoxycarbonyl-Asn(triphénylméthyl)-O-3,4-dihydro-3-hydroxy-4-oxo-1,2,3-benzotriazinyle selon la revendication 1.

20. 9-fluorénylméthoxycarbonyl-Gln(triphénylméthyl)-O-3,4-dihydro-3-hydroxy-4-oxo-1,2,3-benzotriazinyle selon la revendication 1.

21. Une substance gui contient au moins un radical bivalent de formule VII, 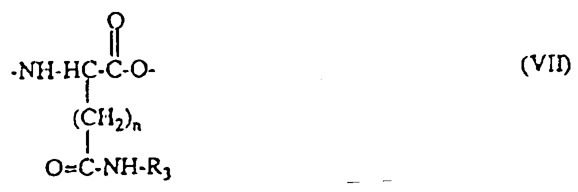 dans laquelle n désigne 1 ou 2 et R₃ désigne un radical triphénylméthyle, 4-monométhoxytrityle ou 4,4'-diméthoxytrityle.

22. Procédé de préparation d'un composé de formule I, 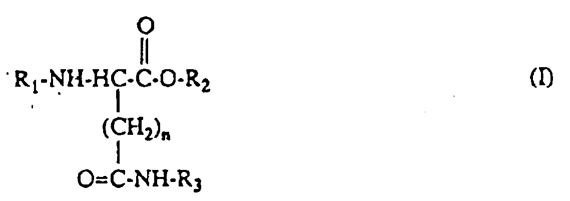 dans laquelle n désigne 1 ou 2, R₁ désigne l'hydrogène ou un groupe de protection de la fonction amino, R₂ désigne l'hydrogène ou un groupe de protection de la fonction carboxyle et R₃ désigne un radical triphénylméthyle, 4-monométhoxytrityle ou 4,4'-diméthoxytrityle, d'un de ses sels dans lequel R₂ désigne l'hydrogène avec un groupe formateur de sel ou un de ses dérivés réactifs d'acide carboxylique dans lequel R₂ désigne l'hydrogène et, par ailleurs, R₁ désigne un groupe de protection de la fonction amino, caractérisé en ce que
a) un composé de formule II, 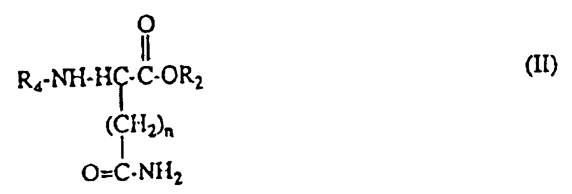 dans laquelle R₄ désigne l'hydrogéne ou un groupe de protection de la fonction amino stable dans les conditions de réaction et n et R₂ ont les significations susmentionnées est amené à réagir avec un composé de formule III,
R₃-OH (III)
dans laquelle R₃ a la signification susmentionnée et en ce que les groupes de protection qui ne sont pas des constituants du produit final souhaité de formule I sont scindés du produit obtenu, ou
b) un groupe de protection de la fonction amino est introduit dans un composé de formule IV, 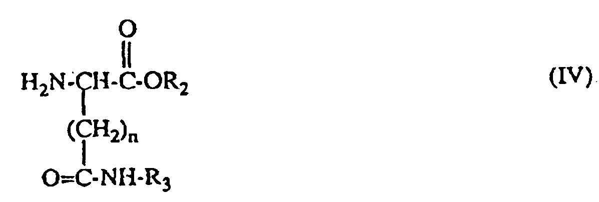 dans laquelle n, R₂ et R₃ ont les significations susmentionnées, pour la préparation d'un composé de formule I dans laquelle R₁ désigne un groupe de protection de la fonction amino, ou
c) un composé de formule V, 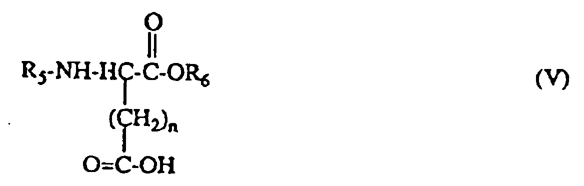 dans laquelle n a la signification susmentionnée, R₅ désigne un groupe de protection de la fonction amino stable dans les conditions de réaction et R₆ désigne un groupe de protection de la fonction carboxyle stable dans les conditions de réaction est amené à réagir avec un composé de formule VI,
R₃-NH₂ (VI)
dans laquelle R₃ a la signification susmentionnée et en ce que les groupes de protection qui ne sont pas des constituants du produit final souhaité de formule I sont scindés du produit obtenu,
et, si souhaité, un composé de formule I obtenu après exécution d'un des procédés a), b) ou c) est transformé en son sel avec un groupe formateur de sel ou un sel obtenu est transformé en son composé libre ou encore un composé obtenu de formule I dans laquelle R₂ désigne l'hydrogène et R₁ désigne un groupe de protection de la fonction amino est transformé en un dérivé réactif d'acide carboxylique.

23. Procédé selon la revendication 22 caractérisé en ce qu'un composé de formule II dans laquelle R₄ désigne un radical benzyloxycarbonyle ou 9-fluorénylméthoxycarbonyle est amené à réagir à une température de l'ordre de +20°C à +70°C dans un solvant organique approprié en présence de quantités catalytiques d'un acide de Lewis fort anhydre et en présence d'un agent déshydratant.

24. Mise en oeuvre d'un composé de formule I dans laquelle R₂ désigne l'hydrogène ou d'un de ses dérivés réactifs d'acide carboxylique selon une des revendications 1 à 20 pour la synthèse des peptides en phase solide.

## Revendications (Revendications pour l'(les) Etat(s) contractant(s) suivant(s): ES)

1. Procédé de préparation d'un composé de formule I, 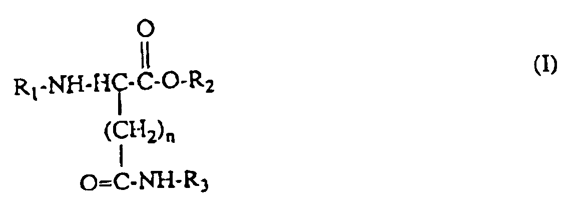 dans laquelle n désigne 1 ou 2, R₁ désigne l'hydrogène ou un groupe de protection de la fonction amino, R₂ désigne l'hydrogène ou un groupe de protection de la fonction carboxyle et R₃ désigne un radical triphénylméthyle, 4-monométhoxytrityle ou 4,4'-diméthoxytrityle, un sel d'un tel composé dans lequel R₂ désigne l'hydrogène avec un groupe formateur de sel ou un dérivé réactif d'acide carboxylique d'un tel composé de formule I, dans laquelle R₂ désigne l'hydrogène et, en outre, R₁ est un groupe de protection de la fonction amino, caractérisé en ce que
a) un composé de formule II, 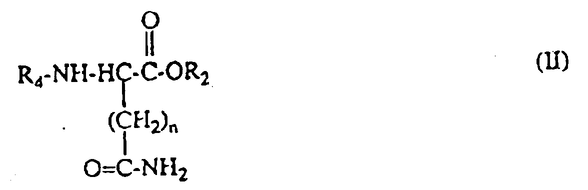 dans laquelle R₄ désigne l'hydrogène ou un groupe de protection de la fonction amino stable dans les conditions de réaction et n et R₂ ont les significations susmentionnées est amené à réagir avec un composé de formule III,
R₃-OH (III)
dans laquelle R₃ a la signification susmentionnée et en ce que les groupes de protection qui ne sont pas des constituants du produit final souhaité de formule I sont scindés du produit obtenu, ou
b) un groupe de protection de la fonction amino est introduit dans un composé de formule IV, 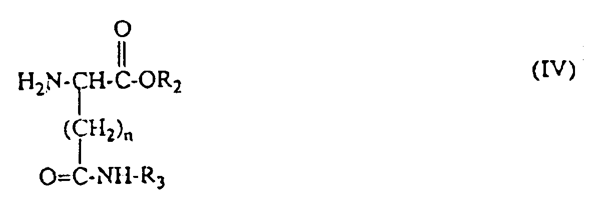 dans laquelle n, R₂ et R₃ ont les significations susmentionnées, pour la préparation d'un composé de formule I dans laquelle R₁ désigne un groupe de protection de la fonction amino, ou
c) un composé de formule V, 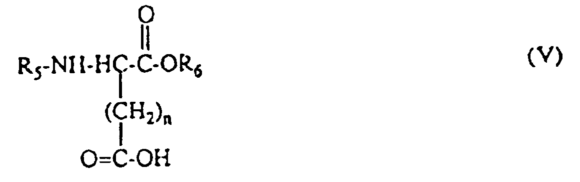 dans laquelle n a la signification susmentionnée, R₅ désigne un groupe de protection de la fonction amino stable dans les conditions de réaction et R₆ désigne un groupe de protection de la fonction carboxyle stable dans les conditions de réaction est amené à réagir avec un composé de formule VI,
R₃-NH₂ (VI)
dans laquelle R₃ a la signification susmentionnée et en ce que les groupes de protection qui ne sont pas des constituants du produit final souhaité de formule I sont scindés du produit obtenu,
et, si souhaité, un composé de formule I obtenu après exécution d'un des procédés a), b) ou c) est transformé en son sel avec un groupe formateur de sel ou un sel obtenu est transformé en son composé libre ou encore un composé obtenu de formule I dans laquelle R₂ désigne l'hydrogène et R₁ désigne un groupe de protection de la fonction amino est transformé en un dérivé réactif d'acide carboxylique.

2. Procédé selon la revendication 1 caractérisé en ce qu'un composé de formule II dans laquelle R₄ désigne un radical benzyloxycarbonyle ou 9-fluorénylméthoxycarbonyle est amené à réagir à une température de l'ordre de +20°C à +70°C dans un solvant organique approprié en présence de quantités catalytiques d'un acide de Lewis fort anhydre et en présence d'un agent déshydratant.

3. Procédé selon la revendication 1 ou 2 de préparation d'un composé de formule I dans laquelle R₁ désigne un groupe de protection de la fonction amino, R₂ désigne l'hydrogène et R₃ désigne un radical triphénylméthyle, 4-monométhoxytrityle ou 4,4'-diméthoxytrityle ou un sel de métal ou d'ammonium de celui-ci.

4. Procédé selon la revendication 1 ou 2 de préparation d'un composé de formule I dans laquelle R₁ désigne un radical benzyloxycarbonyle, 9-fluorénylméthoxycarbonyle, allyloxycarbonyle ou tert.-butyloxycarbonyle, R₂ désigne l'hydrogène et R₃ désigne un radical triphénylméthyle ou un sel de métal ou d'ammonium de celui-ci.

5. Procédé selon la revendication 1 ou 2 de préparation d'un composé de formule I dans laquelle R₁ désigne un radical 9-fluorénylméthoxycarbonyle ou un sel de métal ou d'ammonium d'un tel composé dans lequel R₂ désigne l'hydrogène.

6. Procédé selon la revendication 1 ou 2 de préparation de benzyloxycarbonyl-Asn(triphénylméthyl)-OH ou d'un de ses sels de métal ou d'ammonium.

7. Procédé selon la revendication 1 ou 2 de préparation de 9-fluorénylméthoxycarbonyl-Asn(triphénylméthyl)-OH ou d'un de ses sels de métal ou d'ammonium.

8. Procédé selon la revendication 1 ou 2 de préparation de H-Asn(triphénylméthyl)-OH ou d'un de ses sels.

9. Procédé selon la revendication 1 ou 2 de préparation de tert.-butyloxycarbonyl-Asn(triphénylméthyl)-OH ou d'un de ses sels.

10. Procédé selon la revendication 1 ou 2 de préparation de benzyloxycarbonyl-Gln(triphénylméthyl)-OH ou d'un de ses sels de métal ou d'ammonium.

11. Procédé selon la revendication 1 ou 2 de préparation de 9-fluorénylméthoxycarbonyl-Gln(triphénylméthyl)-OH ou d'un de ses sels de métal ou d'ammonium.

12. Procédé selon la revendication 1 ou 2 de préparation de H-Gln(triphénylméthyl)-OH ou d'un de ses sels.

13. Procédé selon la revendication 1 ou 2 de préparation de tert.-butyloxycarbonyl-Gln(triphénylméthyl)-OH ou d'un de ses sels.

14. Procédé selon la revendication 1 ou 2 de préparation d'un dérivé réactif d'acide carboxylique d'un composé de formule I.

15. Procédé selon la revendication 1 ou 2 de préparation d'un dérivé réactif d'acide carboxylique d'un composé de formule I sous la forme d'un 2,4,5-trichlorophénylester, d'un pentafluorophénylester, d'un chlorure d'acide, d'un anhydride symétrique, d'un ester avec le 1-hydroxybenzotriazole ou d'un ester avec la 3,4-dihydro-3-hydroxy-4-oxo-1,2,3-benzotriazine ou d'un ester avec le N-hydroxy-5-norbornène-2,3-dicarboximide.

16. Procédé selon la revendication 1 ou 2 de préparation de 9-fluorénylméthoxycarbonyl-Asn(triphénylméthyl)-O-2,4,5-trichlorophényle.

17. Procédé selon la revendication 1 ou 2 de préparation de 9-fluorénylméthoxycarbonyl-Gln(triphénylméthyl)-O-2,4,5-trichlorophényle.

18. Procédé selon la revendication 1 ou 2 de préparation de 9-fluorénylméthoxycarbonyl-Asn(triphénylméthyl)-O-pentafluorophényle.

19. Procédé selon la revendication 1 ou 2 de préparation de 9-fluorénylméthoxycarbonyl-Gln(triphénylméthyl)-O-pentafluorophényle.

20. Procédé selon la revendication 1 ou 2 de préparation de 9-fluorénylméthoxycarbonyl-Asn(triphénylméthyl)-O-3,4-dihydro-3-hydroxy-4-oxo-1,2,3-benzotriazinyle.

21. Procédé selon la revendication 1 ou 2 de préparation de 9-fluorénylméthoxycarbonyl-Gln(triphénylméthyl)-O-3,4-dihydro-3-hydroxy-4-oxo-1,2,3-benzotriazinyle.

22. Procédé de préparation d'une substance qui contient au moins un radical bivalent de formule VII, 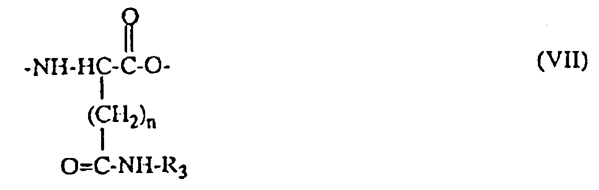 dans laquelle n désigne 1 ou 2 et R₃ désigne un radical triphénylméthyle, 4-monométhoxytrityle ou 4,4'-diméthoxytrityle par synthèse de peptides sur phase solide.
